# EUROPEAN PATENT APPLICATION

(11) **EP 4 592 322 A1**
(43) Date of publication of application: **30.07.2025**
(21) Application number: 22959175.5
(22) Date of filing: 23.09.2022
(51) Int. Cl.: C07K 19/00, C07K 14/46, C07K 14/745, C07K 7/04, C12N 9/64, A61K 38/16, A61P 7/02

(54) **FUSION PROTEIN TARGETING INTERGRIN ALPHA(IIB)BETA3 AND CONTAINING TISSUE PLASMINOGEN ACTIVATOR OR VARIANT THEREOF AND USE THEREOF**

(71) Applicant: Chuang, Woei-jer, Tainan, Taiwan 701 (TW)
(72) Inventor: Chuang, Woei-jer, Tainan, Taiwan 701 (TW)
(74) Representative: Cabinet Chaillot
(86) International application number: PCT/CN2022/120702
(87) International publication number: WO 2024/060167

(57) **Abstract**

The present invention provides a fusion protein, which comprises: a tissue plasminogen activator or a variant thereof; a disintegrin or a variant thereof; and a connector. The connector connects the tissue plasminogen activator or the variant thereof, and the disintegrin or the variant thereof, and comprises an amino acid sequence set forth in any one of SEQ ID NOs: 1 to 7. The present invention further provides a method for treating or preventing thrombosis-related diseases by using the described fusion protein.

## Description

### FIELD OF THE INVENTION

The present invention is directed to a fusion protein and more particularly to a fusion protein containing tissue plasminogen activator or its variant and targeting integrin alpha(IIb)beta(3) and the application of the same protein, including a method for treating or preventing a disease related to thrombosis.

### BACKGROUND OF THE INVENTION

A thrombus is a blood clot caused by the abnormal aggregation of platelet and fibrin in a blood vessel, which can interfere with or block a blood flow in the circulation system to lead to the abnormal blood supply to each body part and further effects on the normal physiological function. Myocardial infarction, cerebral embolism, pulmonary thrombosis, deep vein thrombosis, and peripheral vascular embolism are common disease caused by a thrombus. It is a disease that seriously harms human health and has a high morbidity, a high disability, and a high mortality rate. According to the statistics of the World Health Organization, the number of people who die from thrombosis in the world is about 26 million each year, which is far larger than that from other causes of death.

According to mechanism, drugs for reducing a thrombus are divided into: an antiplatelet agent, an anticoagulant, and a thrombolytic agent. An antiplatelet agent can prevent the platelet aggregation from forming a blood clot, and the common example is aspirin, clopidogrel, or ticagrelor. An anticoagulant can interfere with blood proteins to prolong the clotting time, and the common example is warfarin, rivaroxaban, or heparin. A thrombolytic agent can dissolve a blood clot, and the common example is streptokinase, urokinase, or tissue plasminogen activator (tPA). Although these drugs can be used in the treatment of a thrombus, it may be accompanied by bleeding side effect, leading to other serious disease related to bleeding, e.g., intraparenchymal hemorrhage. Therefore, the related researchers are currently dedicated to strike a balance between reducing a thrombus and overcoming a bleeding problem.

Tissue plasminogen activator is a serine protease, which can bind to fibrin and convert plasminogen into plasmin, the major enzyme of dissolving a blood clot. Genetic engineering is developed to achieve tissue plasminogen activator production in labs, and this genetic engineering product is also called "recombinant tissue plasminogen activator (rtPA)". This recombinant product has undergone various modification to amplify its pharmacokinetic and pharmacodynamic, especially prolonging its short half-life in the circulation and further increasing its fibrin specificity to prevent an unwanted fibrinolytic state. The common recombinant drug, for example, is alteplase, reteplase, or tenecteplase (TNK).

Alteplase presents a protein sequence identical to that of wild-type tissue plasminogen activator produced by human umbilical vein endothelial cells, and is produced through expression in Chinese hamster ovary (CHO) cells. Alteplase has a half-life approximately of 5 minutes, and is suitable for treating an indication of ischemic stroke, ST-elevation myocardial infarction, or acute massive pulmonary embolism. Additionally, alteplase also can manage a patient with a central venous access device (CVAD).

Reteplase is a non-glycosylated form of recombinant tissue plasminogen activator, which presents 355 of the original amino acid residues and is obtained through expression in *Escherichia coli.* Reteplase has a half-life approximately of 14-18 minutes, longer than alteplase, and therefore is allowed to be given as a bolus injection rather than through an infusion like alteplase. Reteplase is suitable for treating an indication of acute myocardial infarction.

Tenecteplase is another modified form of recombinant tissue plasminogen activator and obtained through expression in mammal cells (e.g., CHO cells). Tenecteplase is a glycoprotein presenting 527 amino acid residues, and developed by the modification to cDNA for wild-type human tissue plasminogen activator: a substitution of Threonine at position 103 with Asparagine, a substitution of Asparagine at position 117 with Glutamine, and a tetra-Alanine substitution at positions 296-299. The former two modifications take place at the kringle domain, and the latter one takes place at the protease domain. Among all recombinant tissue plasminogen activators, tenecteplase has the most half-life approximately of 20-24 minutes and is suitable for treating an indication of acute myocardial infarction or pulmonary embolism.

Accordingly, there is a need to develop a novel thrombolytic agent, which can reduce a thrombus and lead to a low bleeding risk.

### SUMMARY OF THE INVENTION

The present invention is made based on the discovery that a fusion protein comprising a tissue plasminogen activator and a disintegrin has a lower inhibitory activity against platelet aggregation than that merely provided by a disintegrin and also has a thrombolytic activity nearly similar with that merely provided by a tissue plasminogen activator. As such, herein is provided a candidate thrombolytic agent which can reduce a thrombus and lead to a low bleeding risk.

Therefore, the present invention provides a fusion protein, which includes: (a) a tissue plasminogen activator or a variant thereof; (b) a disintegrin or a variant thereof; and (c) a linker positioned between the tissue plasminogen activator or the variant thereof and the disintegrin or the variant thereof and the linker having an amino acid sequence selected from SEQ ID Nos.: 1-7.

Exemplarily, a C-terminus of the tissue plasminogen activator or the variant thereof is linked to a N-terminus of the linker, and a N-terminus of the disintegrin or the variant thereof is linked to a C-terminus of the linker.

Exemplarily, a C-terminus of the disintegrin or the variant thereof is linked to a N-terminus of the linker, and a N-terminus of the tissue plasminogen activator or the variant thereof is linked to a C-terminus of the linker.

Exemplarily, the tissue plasminogen activator is alteplase, reteplase, or tenecteplase.

Exemplarily, the tissue plasminogen activator is tenecteplase.

Exemplarily, the tissue plasminogen activator has an amino acid sequence selected from SEQ ID Nos.: 8-10.

Exemplarily, the tissue plasminogen activator has an amino acid sequence of SEQ ID No.: 10.

Exemplarily, the disintegrin is albolabrin, applagin, basilicin, batroxostatin, bitistatin, cereberin, cerastin, crotatroxin, durissin, elegantin, eristicophin, flavoridin, flavostatin, halysin, halystatin, jararacin, jarastatin, kistrin, lachesin, lutosin, molossin, rhodostomin, salmosin, saxatilin, tergeminin, trimestatin, trimucrin, trimutase, ussuristatin, or viridian.

Exemplarily, the disintegrin is rhodostomin or trimucrin.

Exemplarily, the disintegrin variant includes: (a) a linking region having an amino acid sequence selected from SEQ ID Nos.: 11-15; (b) a RGD motif having an amino acid sequence selected from SEQ ID Nos.: 16-28; and (c) a C-terminal region having an amino acid sequence selected from SEQ ID Nos.: 29-33.

Exemplarily, the linking region has an amino acid sequence of SEQ ID No.: 11 or 15.

Exemplarily, the linking region has an amino acid sequence of SEQ ID No.: 11.

Exemplarily, the RGD motif has an amino acid sequence selected from SEQ ID Nos.: 17-21 and 24-26.

Exemplarily, the RGD motif has an amino acid sequence of SEQ ID No.: 20.

Exemplarily, the C-terminal region has an amino acid sequence of SEQ ID No.: 31.

Exemplarily, the disintegrin variant has an amino acid sequence of SEQ ID No.: 34.

Exemplarily, the fusion protein has an amino acid sequence selected from SEQ ID Nos.: 35-42.

Exemplarily, the fusion protein is provided for targeting integrin alpha(IIb)beta(3) and fibrin.

Since fibrin is the major component of blood clots, the fusion protein herein can bind to fibrin in a thrombus and convert plasminogen into plasmin. In such a way, plasmin can degrade fibrin, resulting in fibrin degradation products (FDPs), to achieve thrombolysis. Additionally, since integrin alpha(IIb)beta(3) is expressed at a high level in platelets and their progenitors (see J Hematol Oncol. 2019 Mar 7;12(1):26), the fusion protein herein can also bind to platelet integrin alpha(IIb)beta(3) in a thrombus to inhibit platelet aggregation so as to avoid large thrombus reformation. Moreover, the fusion protein herein has an inhibitory activity against platelet aggregation less than that of a disintegrin or its variant so that the bleeding risk can reduce. Therefore, the fusion protein herein can reduce a thrombus and lead to a low bleeding risk. Based on this, the fusion protein herein has the potential to become a clinically-available thrombolytic agent.

The present invention further provides a pharmaceutical composition, which includes: the foregoing fusion protein; and a pharmaceutically acceptable carrier.

Exemplarily, the pharmaceutical composition is an orally administrable formulation, an injectable formulation, an inhalable formulation, or a topically or transdermally administrable formulation.

Exemplarily, based on total volume of the pharmaceutical composition, the fusion protein has a molar concentration of 1-1400 nM.

The present invention further provides use of the foregoing pharmaceutical composition, which is for manufacturing a medicine for treating or preventing a disease related to thrombosis under the condition of a low bleeding risk.

Exemplarily, the disease related to thrombosis is venous thrombosis or arterial thrombosis.

Exemplarily, the venous thrombosis is branch retinal vein occlusion, Budd-Chiari syndrome, cavernous sinus thrombosis, central retinal vein occlusion, cerebral venous sinus thrombosis, deep vein thrombosis, jugular vein thrombosis, mesenteric vein thrombosis, Paget-Schroetter disease, parodoxical embolism, portal vein thrombosis, pulmonary embolism, renal vein thrombosis, or splenic vein thrombosis.

Exemplarily, the arterial thrombosis is hepatic artery thrombosis, limb ischemia, myocardial infarction, or stroke.

The present invention further provides a method for treating or preventing a disease related to thrombosis, which includes: administering the foregoing pharmaceutical composition to a subject in need thereof to dissolve a thrombus and to lead to a low bleeding risk.

Exemplarily, the disease related to thrombosis is venous thrombosis or arterial thrombosis.

Exemplarily, the venous thrombosis is branch retinal vein occlusion, Budd-Chiari syndrome, cavernous sinus thrombosis, central retinal vein occlusion, cerebral venous sinus thrombosis, deep vein thrombosis, jugular vein thrombosis, mesenteric vein thrombosis, Paget-Schroetter disease, parodoxical embolism, portal vein thrombosis, pulmonary embolism, renal vein thrombosis, or splenic vein thrombosis.

Exemplarily, the arterial thrombosis is hepatic artery thrombosis, limb ischemia, myocardial infarction, or stroke.

Exemplarily, the fusion protein is administered to the subject in a dose of 0.1-1000 mg/kg body weight of the subject.

The present invention further provides a nucleic acid, which includes a nucleotide sequence for encoding the foregoing fusion protein.

The present invention further provides a host cell, which has the foregoing nucleic acid.

Exemplarily, the host cell is a prokaryotic cell or a eukaryotic cell.

Exemplarily, the prokaryotic cell is *Escherichia coli.*

Exemplarily, the eukaryotic cell is a CHO cell, a COS cell, or a HEK293 cell.

The present invention further provides a method for producing the foregoing fusion protein, which includes: incubating the foregoing host cell to express the fusion protein.

### BRIEF DESCRIPTION OF THE DRAWINGS

FIG. 1 is a flow diagram showing the production of a DNA construct for expressing protein TNK-G₉-RR;
FIG. 2A is a liquid chromatogram showing the isolation result of protein TNK;
FIG. 2B is a liquid chromatogram showing the isolation result of protein TNK-G₉-RR;
FIG. 2C is a liquid chromatogram showing the isolation result of protein TNK-(G₄S)₃-RR;
FIG. 2D is a liquid chromatogram showing the isolation result of protein TNK-(PA)₃-RR;
FIG. 2E is a liquid chromatogram showing the isolation result of protein TNK-(PA)₅-RR;
FIG. 2F is a liquid chromatogram showing the isolation result of protein TNK-(PA)₇-RR;
FIG. 2G is a liquid chromatogram showing the isolation result of protein TNK-EA₃K(G₄S)₂-RR;
FIG. 2H is a liquid chromatogram showing the isolation result of protein TNK-(EA₃K)₃-RR;
FIG. 2I is a liquid chromatogram showing the isolation result of protein RR-(PA)₃-TNK;
FIGS. 3A-3B are respectively a non-reducing Tris-glycine SDS-PAGE picture and a reducing Tris-glycine SDS-PAGE picture illustrating the isolation result of protein TNK; wherein the symbol "M" stands for a protein marker, the symbol "LS" stands for a loading sample (20 µL), the symbol "FT" stands for a flow-through sample (250 µL), and each Arabic numeral stands for the number of the corresponding fraction;
FIGS. 3C-3D are respectively a non-reducing Tris-glycine SDS-PAGE picture and a reducing Tris-glycine SDS-PAGE picture illustrating the isolation result of protein TNK-G₉-RR; wherein the symbol "M" stands for a protein marker, the symbol "LS" stands for a loading sample (20 µL), the symbol "FT" stands for a flow-through sample (250 µL), and each Arabic numeral stands for the number of the corresponding fraction;
FIGS. 3E-3F are respectively a non-reducing Tris-glycine SDS-PAGE picture and a reducing Tris-glycine SDS-PAGE picture illustrating the isolation result of protein TNK-(G₄S)₃-RR; wherein the symbol "M" stands for a protein marker, the symbol "LS" stands for a loading sample (20 µL), the symbol "FT" stands for a flow-through sample (250 µL), and each Arabic numeral stands for the number of the corresponding fraction;
FIGS. 3G-3H are respectively a non-reducing Tris-glycine SDS-PAGE picture and a reducing Tris-glycine SDS-PAGE picture illustrating the isolation result of protein TNK-(PA)₃-RR; wherein the symbol "M" stands for a protein marker, the symbol "LS" stands for a loading sample (20 µL), the symbol "FT" stands for a flow-through sample (250 µL), and each Arabic numeral stands for the number of the corresponding fraction;
FIGS. 3I-3J are respectively a non-reducing Tris-glycine SDS-PAGE picture and a reducing Tris-glycine SDS-PAGE picture illustrating the isolation result of protein TNK-(PA)₅-RR; wherein the symbol "M" stands for a protein marker, the symbol "LS" stands for a loading sample (20 µL), the symbol "FT" stands for a flow-through sample (250 µL), and each Arabic numeral stands for the number of the corresponding fraction;
FIGS. 3K-3L are respectively a non-reducing Tris-glycine SDS-PAGE picture and a reducing Tris-glycine SDS-PAGE picture illustrating the isolation result of protein TNK-(PA)₇-RR; wherein the symbol "M" stands for a protein marker, the symbol "LS" stands for a loading sample (20 µL), the symbol "FT" stands for a flow-through sample (250 µL), and each Arabic numeral stands for the number of the corresponding fraction;
FIGS. 3M-3N are respectively a non-reducing Tris-glycine SDS-PAGE picture and a reducing Tris-glycine SDS-PAGE picture illustrating the isolation result of protein TNK-EA₃K(G₄S)₂-RR; wherein the symbol "M" stands for a protein marker, the symbol "LS" stands for a loading sample (20 µL), the symbol "FT" stands for a flow-through sample (250 µL), and each Arabic numeral stands for the number of the corresponding fraction;
FIGS. 3O-3P are respectively a non-reducing Tris-glycine SDS-PAGE picture and a reducing Tris-glycine SDS-PAGE picture illustrating the isolation result of protein TNK-(EA₃K)₃-RR; wherein the symbol "M" stands for a protein marker, the symbol "LS" stands for a loading sample (20 µL), the symbol "FT" stands for a flow-through sample (250 µL), and each Arabic numeral stands for the number of the corresponding fraction;
FIGS. 3Q-3R are respectively a non-reducing Tris-glycine SDS-PAGE picture and a reducing Tris-glycine SDS-PAGE picture illustrating the isolation result of protein RR-(PA)₃-TNK; wherein the symbol "M" stands for a protein marker, the symbol "LS" stands for a loading sample (20 µL), the symbol "FT" stands for a flow-through sample (250 µL), and each Arabic numeral stands for the number of the corresponding fraction;
FIG. 4A is a curve chart illustrating the thrombolysis rates over time of protein TNK at different concentrations;
FIG. 4B is a curve chart illustrating the thrombolysis rates over time of protein TNK-G₉-RR at different concentrations;
FIG. 4C is a curve chart illustrating the thrombolysis rates over time of protein TNK-(G₄S)₃-RR at different concentrations;
FIG. 4D is a curve chart illustrating the thrombolysis rates over time of protein TNK-(PA)₃-RR at different concentrations;
FIG. 4E is a curve chart illustrating the thrombolysis rates over time of protein TNK-(PA)₅-RR at different concentrations;
FIG. 4F is a curve chart illustrating the thrombolysis rates over time of protein TNK-(PA)₇-RR at different concentrations;
FIG. 4G is a curve chart illustrating the thrombolysis rates over time of protein TNK-EA₃K(G₄S)₂-RR at different concentrations;
FIG. 4H is a curve chart illustrating the thrombolysis rates over time of protein TNK-(EA₃K)₃-RR at different concentrations;
FIG. 4I is a curve chart illustrating the thrombolysis rates over time of protein RR-(PA)₃-TNK at different concentrations; and
FIG. 5 is a bar chart illustrating the time to 50% thrombus lysis of different proteins at a concentration of 7.0 nM.

### DETAILED DESCRIPTION OF THE INVENTION

The detailed description and preferred embodiments of the invention will be set forth in the following content, and provided for people skilled in the art to understand the characteristics of the invention.

### 1. DEFINITIONS

The term "protein" used herein includes a wild-type protein produced by a natural cell, a recombinant protein made through genetic engineering, or a synthetic protein made through chemical synthesis, unless otherwise provided. Upon the condition of no adverse effect on the original activity, substitution, deletion, and/or insertion of at least one amino acid may be included in the protein sequence.

The term "amino acid" used herein includes: D-amino acid or L-amino acid, unless otherwise provided. "D-" and "L-" are used to refer to absolute configuration of the amino acid, rather than a particular rotation direction of plane-polarized light. In the present disclosure, amino acid may be referred to by the one-letter symbol recommended by the IUPAC-IUB Biochemical Nomenclature Commission, unless otherwise provided. A character string composed of multiple one-letter symbols is used to represent a protein sequence, and the order of one-letter symbol in the protein sequence corresponds to the direction from the N-terminus to the C-terminus of amino acid in the protein. If there is a superscript deposited in front of a one-letter symbol, it indicates the number of the corresponding amino acid residue from the N-terminus. For example, ⁶⁷PRNGLYG indicate that Proline is at position 67 from the N-terminus of a protein, and so on. If there is a subscript deposited behind a one-letter symbol, it indicates the number of repeating of the corresponding amino acid residue or the corresponding amino acid unit. For example, G₉ indicates that 9 consecutive Glycine residues are connected, and so on; for example, (G₄S)₃ indicates that 3 consecutive amino acid units composed of Glycine-Glycine-Glycine-Glycine-Serine are connected, and so on.

Substitution, deletion, and/or insertion in a protein sequence may occur in the protein non-functional domain, which usually has no effect on the original activity. Additionally, amino acid substitution may include conservative amino acid substitution, and conservative amino acid substitution indicates substitution within amino acid residues having a similar characteristic or within amino acid residues having a related side-chain. Substitution within amino acid residues having a similar characteristic, for example, is substitution within acidic amino acid residues, i.e., Aspartate and Glutamate, substitution within basic amino acid residues, i.e., Lysine, Arginine, and Histidine, substitution within non-polar amino acid residues, i.e., Alanine, Valine, Leucine, Isoleucine, Proline, Phenylalanine, Methionine, and Tryptophan, or substitution within uncharged amino acid residues, i.e., Glycine, Asparagine, Glutamine, Cysteine, Serine, Threonine, and Tyrosine. Substitution within amino acid residues having a related side-chain, for example, is substitution within aliphatic-hydroxy amino acid residues, i.e., Serine and Threonine, substitution within amide-containing amino acid residues, i.e., Asparagine and Glutamine, substitution within aliphatic amino acid residues, i.e., Alanine, Valine, Leucine, and Isoleucine, or substitution within aromatic amino acid residues, i.e., Phenylalanine, Tryptophan, and Tyrosine.

The term "tissue plasminogen activator" used herein includes a wild-type tissue plasminogen activator or a recombinant tissue plasminogen activator, unless otherwise provided. The wild-type form, for example, is a tissue plasminogen activator produced by human vascular endothelial cells; the recombinant form, for example, is alteplase (SEQ ID No.: 8), reteplase (SEQ ID No.: 9), or tenecteplase (SEQ ID No.: 10).

The term "disintegrin" used herein is a platelet aggregation inhibitor collected from viper's saliva, and usually has 47-84 amino acid residues and 4-7 disulfide bonds, unless otherwise provided. The disintegrin, for example, is albolabrin, applagin, basilicin, batroxostatin, bitistatin, cereberin, cerastin, crotatroxin, durissin, elegantin, eristicophin, flavoridin, flavostatin, halysin, halystatin, jararacin, jarastatin, kistrin, lachesin, lutosin, molossin, rhodostomin, salmosin, saxatilin, tergeminin, trimestatin, trimucrin, trimutase, ussuristatin, or viridian.

The term "variant" used herein is a modified protein obtained through substitution, deletion, and/or insertion of at least one amino acid in a reference protein sequence without having effects on the original activity, unless otherwise provided. For example, a disintegrin variant includes a rhodostomin variant or a trimucrin variant, which can bind to integrin alpha(IIb)beta(3) to inhibit platelet aggregation like a wild-type disintegrin. A variant may have an at least 95%, 90%, 85%, 80%, 75%, 70%, 65%, or 60% sequence identity to a reference sequence, and the sequence identity is defined as a gap-excluded identity, a BLAST identity, or a gap-compressed identity. A BLAST identity may be obtained via the basic local alignment search tool offered by the National Center for Biotechnology Information. For example, the trimucrin variant RR used herein has a 95% identity to the wild-type trimucrin.

The term "RGD motif" used herein is a flexible loop composed of Arginine-Glycine-Aspartate in a disintegrin, which is an integrin-binding domain, unless otherwise provided. For example, the RGD motif of wild-type trimucrin comprises ⁵⁰ARGDNP, and that of wild-type rhodostomin comprises ⁴⁸PRGDMP. With reference to the mentioned above, the RGD motif of variant trimucrin may has at least one mutant amino acid residue at the RGD motif ⁵⁰ARGDNP of wild-type trimucrin without effect on the original functions, e.g., SEQ ID No.: 16, 17, 18, 19, 20, 21, 22, 23, 24, 25, or 26; the RGD motif of variant rhodostomin may has at least one mutant amino acid residue at the RGD motif ⁴⁸PRGDMP of wild-type rhodostomin without effect on the original functions, e.g., SEQ ID No.: 27, or 28.

The term "linking region" used herein is a region adjacent to a N-terminus of the RGD motif in the disintegrin and is usually an amino acid fragment at positions 38-49, unless otherwise provided. For example, the linking region of wild-type trimucrin comprises ⁴¹KKKRT (SEQ ID No.: 11), and that of wild-type rhodostomin comprises ³⁹SRAGK (SEQ ID No.: 12). With reference to the mentioned above, the linking region of variant trimucrin may has at least one mutant amino acid residue at the linking region ⁴¹KKKRT of wild-type trimucrin without effect on the original functions, e.g., SEQ ID No.: 13, 14, or 15.

The term "C-terminal region" used herein is a region adjacent to a C-terminus of the RGD motif in the disintegrin, unless otherwise provided. For example, the C-terminal region of wild-type trimucrin comprises ⁶⁷PRNGLYG (SEQ ID No.: 29), and that of wild-type rhodostomin comprises ⁶⁵PRYH (SEQ ID No.: 30). With reference to the mentioned above, the C-terminal region of variant trimucrin may has at least one mutant amino acid residue at the C-terminal region ⁶⁷PRNGLYG of wild-type trimucrin without effect on the original functions, e.g., SEQ ID No.: 31, 32, or 33.

The term "disintegrin variant" used herein comprises at least one mutant amino acid residue at the wild-type RGD motif, at least one mutant amino acid residue at the wild-type linking region, and/or at least one mutant amino acid residue at the wild-type C-terminal region, unless otherwise provided, e.g., SEQ ID No.: 34.

The term "treating" used herein indicates to provide a therapeutic intervention for curing or ameliorating thrombosis disease or disorder, unless otherwise provided. That is, treating comprises completely or almost completely curing or ameliorating the disease or disorder.

The term "preventing" used herein indicates to completely or almost completely stop thrombosis disease or disorder from occurring, unless otherwise provided. For example, when no thrombosis or slight thrombosis but not resulting in disease or disorder occurs in a patient or subject, a preventive intervention is provided to prevent the disease or disorder from occurring.

The term "pharmaceutically acceptable carrier" used herein indicates an additive which is, within the scope of sound medical judgment, suitable for use in contact with the tissue of a subject without excessive toxicity, irritation, allergic response, or other problem or complication, and commensurate with a reasonable benefit/risk ratio, unless otherwise provided, e.g., a filler, a dilutant, an agglutinant, an adhesive, a lubricant, a fluidizer, a stabilizer, a colorant, a humectant, or a disintegrant.

### 2. FUSION PROTEIN

A first embodiment of the present invention provides a fusion protein, which can both bind to fibrin and platelet integrin alpha(IIb)beta(3) in a thrombus. Binding to fibrin can convert plasminogen into plasmin so that plasmin can degrade fibrin into fibrin degradation products to achieve thrombolysis. Moreover, binding to integrin alpha(IIb)beta(3) can inhibit platelet aggregation to stop large thrombus from reforming. However, the inhibitory activity against platelet aggregation caused by binding of the fusion protein according to the first embodiment to integrin alpha(IIb)beta(3) is lower than that caused by binding of the only disintegrin or it variant to integrin alpha(IIb)beta(3) so that the bleeding risk can reduce. Based on the foregoing properties, the fusion protein according to the first embodiment can be used for thrombolysis, resulting in a low bleeding risk; that is, it can be used for treating thrombosis disease or disorder under the condition of a low bleeding risk.

The fusion protein according to the first embodiment comprises: (a) a tissue plasminogen activator or a tissue plasminogen activator variant; (b) a disintegrin or a disintegrin variant; and (c) a linker positioned between the tissue plasminogen activator or the tissue plasminogen activator variant and the disintegrin or the disintegrin variant and the linker having an amino acid sequence of SEQ ID No.: 1, 2, 3, 4, 5, 6, or 7. The fusion protein, from a N-terminus to a C-terminus, may comprises: (a) the tissue plasminogen activator or the tissue plasminogen activator variant; (c) the linker; and (b) the disintegrin or the disintegrin variant; or may comprise: (b) the disintegrin or the disintegrin variant; (c) the linker; and (a) the tissue plasminogen activator or the tissue plasminogen activator variant. Specifically, a C-terminus of the tissue plasminogen activator or its variant may be linked to a N-terminus of the linker, and a N-terminus of the disintegrin or its variant may be linked to a C-terminus of the linker; or a C-terminus of the disintegrin or its variant may be linked to a N-terminus of the linker, and a N-terminus of the tissue plasminogen activator or its variant may be linked to a C-terminus of the linker. Additionally, the term "being connected" is not limited to "directly being connected" or "indirectly being connected"; that is, there is no connecting fragment or at least one connecting fragment positioned between the two connected proteins. Preferably, the fusion protein comprises an amino acid sequence of SEQ ID No.: 35, 36, 37, 38, 39, 40 ,41, or 42.

In term of species, the tissue plasminogen activator may be alteplase, reteplase, or tenecteplase. Preferably, the tissue plasminogen activator is tenecteplase.

In term of sequence, the tissue plasminogen activator may comprise an amino acid sequence of SEQ ID No.: 8, 9, or 10. Preferably, the tissue plasminogen activator comprises an amino acid sequence of SEQ ID No.: 10.

In term of species, the disintegrin may be albolabrin, applagin, basilicin, batroxostatin, bitistatin, cereberin, cerastin, crotatroxin, durissin, elegantin, eristicophin, flavoridin, flavostatin, halysin, halystatin, jararacin, jarastatin, kistrin, lachesin, lutosin, molossin, rhodostomin, salmosin, saxatilin, tergeminin, trimestatin, trimucrin, trimutase, ussuristatin, or viridian. Preferably, the disintegrin is rhodostomin or trimucrin.

In term of sequence, the disintegrin variant may comprise: a linking region, a RGD motif, and a C-terminal region; the linking region comprises an amino acid sequence of SEQ ID No.: 11, 12, 13, 14, or 15, the RGD motif comprises an amino acid sequence of SEQ ID No.: 16, 17, 18, 19, 20, 21, 22, 23, 24, 25, 26, 27, or 28, and the C-terminal region comprises an amino acid sequence of SEQ ID No.: 29, 30, 31, 32, or 33.

Preferably, the linking region comprises an amino acid sequence of SEQ ID No.: 11 or 15. More preferably, the linking region comprises an amino acid sequence of SEQ ID No.: 11.

Preferably, the RGD motif comprises an amino acid sequence of SEQ ID No.: 17, 18, 19, 20, 21, 24, 25, or 26. More preferably, the RGD motif comprises an amino acid sequence of SEQ ID No.: 20.

Preferably, the C-terminal region comprises an amino acid sequence of SEQ ID No.: 31.

Preferably, the disintegrin variant comprises an amino acid sequence of SEQ ID No.: 34.

The fusion protein according to the first embodiment may be produced through genetic engineering or chemical synthesis. The chemical synthesis, for example, is solid-phase synthesis or liquid-phase synthesis. Ammonium sulfate or ethanol precipitation, acid extraction, ion-exchange chromatography, affinity chromatography, or lectin chromatography may be implemented for isolation of purification of the fusion protein according to the first embodiment. Preferably, high performance liquid chromatography (HPLC) is implemented.

The fusion protein according to the first embodiment may further include a hydrophilic group for increasing its solubility or circulation half-life. The hydrophilic group may be connected to a N-terminus of the fusion protein. Preferably, the hydrophilic group is polyethylene glycol, polypropylene glycol, polylactic acid, polyglycolic acid, polyvinyl alcohol, or dextran. More preferably, the hydrophilic group is polyethylene glycol containing 2-20 repeating ethylene glycol units.

The fusion protein according to the first embodiment may further include an affinity tag for protein purification. The affinity tag may be connected to a N-terminus or a C-terminus of the fusion protein. Preferably, the affinity tag is a His-tag, a GST-tag, a MBP-tag, a NusA-tag, or a SUMO-tag.

### 3. PHARMACEUTICAL COMPOSITION

A second embodiment of the present invention provides a pharmaceutical composition, and the pharmaceutical composition includes a fusion protein according to the first embodiment. As such, the pharmaceutical composition can be administered to a subject in need of thrombolysis to realize thrombolysis under the condition of a low bleeding risk. The pharmaceutical composition according to the second embodiment comprises: the fusion protein according to the first embodiment, and a pharmaceutically acceptable carrier.

Generally, the pharmaceutically acceptable carrier can allow the pharmaceutical composition to be in various forms or to be suitable in various routes of administration. Preferably, the pharmaceutical composition is an orally administrable formulation, an injectable formulation, an inhalable formulation, or a topically or transdermally administrable formulation for various routes of administration. Preferably, the pharmaceutical composition is a tablet, a capsule, a granule, a dispersant, a solution, a syrup, a suspension, or an emulsion. The pharmaceutical composition may be also coated on an implantable device, e.g., a stent or a catheter so that thrombolysis is achieved accompanied by preventing restenosis or supporting and reinforcing a vessel wall with the implantable device. The pharmaceutical composition may further comprise another drug for reducing a thrombus, e.g., an antiplatelet agent or an anticoagulant. The antiplatelet agent, for example, is aspirin, clopidogrel, or ticagrelor; the anticoagulant, for example, is warfarin, rivaroxaban, or heparin.

The pharmaceutically acceptable carrier may be an excipient, a filler, a dilutant, an agglutinant, an adhesive, a lubricant, a fluidizer, a stabilizer, a colorant, a humectant, or a disintegrant. The excipient, for example, is sodium citrate, calcium carbonate, or calcium phosphate; the filler, for example, is lactose or high molecular weight polyethylene glycol; the dilutant, for example, is water, ethanol, propanediol, or glycerol; the adhesive, for example, is sucrose, gelatin, or acacia gum; the lubricant, for example, is magnesium stearate, calcium stearate, zinc stearate, sodium stearate, stearate, aluminum stearate, leucine, glycerol behenate, or hydrogenated vegetable oil; the fluidizer, for example, is sodium aluminosilicate, calcium silicate, microcrystalline cellulose, maize starch, sodium benzoate, calcium carbonate, magnesium carbonate, talcum, calcium stearate, magnesium stearate, zinc stearate, magnesium lauryl sulfate, or magnesium oxide; the stabilizer, for example, is citrate or ascorbic acid; the colorant, for example, is titanium dioxide or ferric oxide; the humectant, for example, is Pluronic F68, Tween 20, or Tween 80; the disintegrant, for example, is potato starch, tapioca starch, or silicate.

Based on total volume of the pharmaceutical composition, the fusion protein may have a molar concentration of 1-1400 nM. Preferably, based on the total volume of the pharmaceutical composition, the fusion protein has a molar concentration of 7-1370.7 nM.

### 4. PHARMACEUTICAL USE

A third embodiment of the present invention provides use of a pharmaceutical composition according to the second embodiment, which is for manufacturing a medicine for treating or preventing a disease related to thrombosis, resulting in a low bleeding risk. This medicine can be administered to a subject in need of thrombolysis to realize thrombolysis under the condition of a low bleeding risk. In other words, this medicine can be administered to a subject in need of treating or preventing a disease related to thrombosis to achieve the treatment or prevention effect under the condition of a low bleeding risk.

This medicine may be administered by different routes, e.g., oral administration, injection administration, inhalation administration, or topical or transdermal administration. Additionally, the fusion protein may be administered to the subject in a dose of 0.1-1000 mg/kg body weight of the subject.

The disease related to thrombosis may be divided into venous thrombosis or arterial thrombosis. The venous thrombosis, for example, is branch retinal vein occlusion, Budd-Chiari syndrome, cavernous sinus thrombosis, central retinal vein occlusion, cerebral venous sinus thrombosis, deep vein thrombosis, jugular vein thrombosis, mesenteric vein thrombosis, Paget-Schroetter disease, parodoxical embolism, portal vein thrombosis, pulmonary embolism, renal vein thrombosis, or splenic vein thrombosis; the arterial thrombosis, for example, is hepatic artery thrombosis, limb ischemia, myocardial infarction, or stroke.

A fourth embodiment of the present invention provides a method for treating or preventing a disease related to thrombosis, which includes: administering a pharmaceutical composition according to the second embodiment to a subject in need of this treatment or this prevention to dissolve a thrombus and to lead to a low bleeding risk.

This pharmaceutical composition may be administered by different routes, e.g., oral administration, injection administration, inhalation administration, or topical or transdermal administration. Additionally, the fusion protein may be administered to the subject in a dose of 0.1-1000 mg/kg body weight of the subject.

The disease related to thrombosis may be divided into venous thrombosis or arterial thrombosis. The venous thrombosis, for example, is branch retinal vein occlusion, Budd-Chiari syndrome, cavernous sinus thrombosis, central retinal vein occlusion, cerebral venous sinus thrombosis, deep vein thrombosis, jugular vein thrombosis, mesenteric vein thrombosis, Paget-Schroetter disease, parodoxical embolism, portal vein thrombosis, pulmonary embolism, renal vein thrombosis, or splenic vein thrombosis; the arterial thrombosis, for example, is hepatic artery thrombosis, limb ischemia, myocardial infarction, or stroke.

### 5. OTHERS

A fifth embodiment of the present invention provides a nucleic acid, which includes a nucleotide sequence for encoding a fusion protein according to the first embodiment. For controlling the protein expression, the nucleic acid may further include a promoter, which is operably linked to the nucleotide sequence for encoding a fusion protein. The term "being operably linked to" herein indicates that two or more than two nucleotide sequences are in a functional relationship with each other.

A sixth embodiment of the present invention provides a host cell, which includes a nucleic acid according to the fifth embodiment. Since the host cell has the nucleotide sequence for encoding the fusion protein, the fusion protein can be produced through cultivating the host cell. The host cell may be a prokaryotic cell or a eukaryotic cell. The prokaryotic cell, for example, is *Escherichia coli;* the eukaryotic cell, for example, is a CHO cell, a COS cell, or a HEK293 cell.

A seventh embodiment of the present invention provides a method for producing a fusion protein according to the first embodiment, which includes: incubating the host cell to express the fusion protein. Further, an inducer may be selected for inducing the host cell to express the protein according to the promoter.

The following examples are offered to further illustrate the invention.

### Example 1: Protein Production

An expression construct was transfected into CHO cells or yeast cells to express a fusion protein. The cell culture supernatant was collected, and then the liquid chromatography was performed to purify the fusion protein.

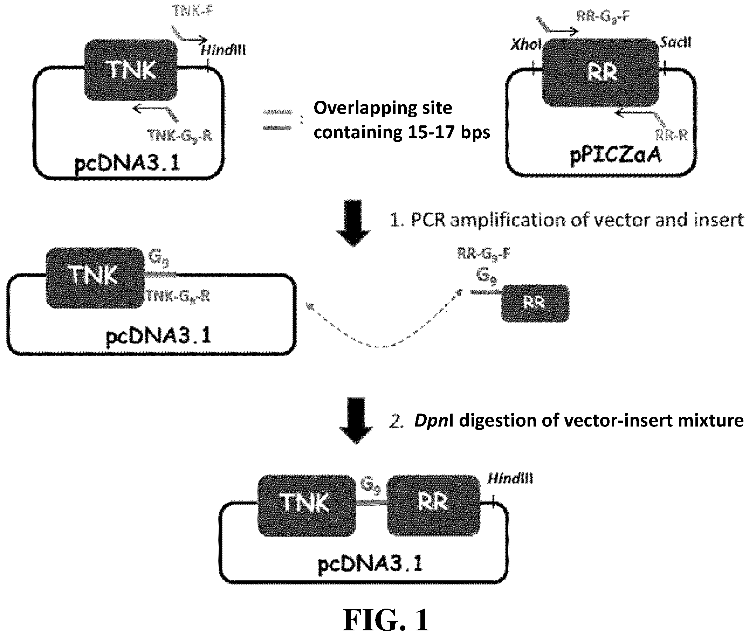

An expression construct for protein TNK-G₉-RR as shown in FIG. 1 is taken as an example. Polymerase chain reaction was performed on a plasmid pcDNA3.1 containing a TNK nucleotide fragment with a primer pair of TNK-F and TNK-G₉-R to obtain a plasmid pcDNA3.1 containing a TNK-G₉ nucleotide fragment; polymerase chain reaction was performed on a plasmid pPICZαA containing a RR nucleotide fragment with a primer pair of RR-G₉-F and RR-R to obtain an insert containing a RR nucleotide fragment. The thus-obtained plasmid pcDNA3.1 containing a TNK-G₉ nucleotide fragment and the thus-obtained insert containing a RR nucleotide fragment were both treated with restriction enzyme *DpnI,* and then the DpnI-treated plasmid pcDNA3.1 and the DpnI-treated insert were joined to obtain a plasmid pcDNA3.1 containing a TNK-G₉-RR nucleotide fragment to be the expression construct for protein TNK-G₉-RR.

A plasmid pcDNA3.1 containing a TNK-(G₄S)₃-RR nucleotide fragment, an expression construct for protein TNK-(G₄S)₃-RR, was obtained following the foregoing procedure except that a primer TNK-(G₄S)₃-R is substituted for the primer TNK-G₉-R, and a primer RR-(G₄S)₃-F is substituted for the primer RR-G₉-F.

A plasmid pcDNA3.1 containing a TNK-(PA)₃-RR nucleotide fragment, an expression construct for protein TNK-(PA)₃-RR, was obtained following the foregoing procedure except that a primer TNK-(PA)₃-R is substituted for the primer TNK-G₉-R, and a primer RR-(PA)₃-F is substituted for the primer RR-G₉-F.

A plasmid pcDNA3.1 containing a TNK-(PA)₃-RR nucleotide fragment, an expression construct for protein TNK-(PA)₅-RR, was obtained following the foregoing procedure except that a primer TNK-(PA)₅-R is substituted for the primer TNK-G₉-R, and a primer RR-(PA)₅-F is substituted for the primer RR-G₉-F.

A plasmid pcDNA3.1 containing a TNK-(PA)₇-RR nucleotide fragment, an expression construct for protein TNK-(PA)₇-RR, was obtained following the foregoing procedure except that a primer TNK-(PA)₇-R is substituted for the primer TNK-G₉-R, and a primer RR-(PA)₇-F is substituted for the primer RR-G₉-F.

A plasmid pcDNA3.1 containing a TNK-EA₃K(G₄S)₂-RR nucleotide fragment, an expression construct for protein TNK-EA₃K(G₄S)₂-RR, was obtained following the foregoing procedure except that a primer TNK-EA₃K(G₄S)₂-R is substituted for the primer TNK-G₉-R, and a primer RR-EA₃K(G₄S)₂-F is substituted for the primer RR-G₉-F.

A plasmid pcDNA3.1 containing a TNK-(EA₃K)₃-RR nucleotide fragment, an expression construct for protein TNK-(EA₃K)₃-RR, was obtained following the foregoing procedure except that a primer TNK-(EA₃K)₃-R is substituted for the primer TNK-G₉-R, and a primer RR-(EA₃K)₃-F is substituted for the primer RR-G₉-F.

The nucleotide sequences of all primers are listed in Table 1.

**Table 1. Primer sequence**

| Primer | Nucleotide sequence (from 5'-end to 3'-end) |
|---|---|
| TNK-F | gacaacatgcgaccgtagaagcttggtaccgagctc |
| RR-R | ccgcggttaaccgtacaatctgtttcttggacagtc |
| TNK-G₉-R | cgtgacaacatgcgaccggggggaggcggaggcggagggggcggagaagcaggt |
| RR-G₉-F | cgtgacaacatgcgaccggggggaggcggaggcggagggggcggagaagcaggtgaagaatg |
| TNK-(G₄S)₃-R | actgcctcctccacctgagcctccccctcccggtcgcatgttgtcac |
| RR-(G₄S)₃-F | ggtggaggaggcagtgggggtggtggatctgaagcaggtgaagaatgtgac |
| TNK-(PA)₃-R | ggctggtgcgggagcaggcggtcgcatgttgtcacga |
| RR-(PA)₃-F | tgctcccgcaccagccgaggcaggtgaagaatgtgactgt |
| TNK-(PA)₅-R | ggctggtgcgggagcaggagctggggcgggcggtcgcatgttgtcacga |
| RR-(PA)₃-F | tgctcccgcaccagccgaggcaggtgaagaatgtgactgt |
| TNK-(PA)₇-R | gctggtgcgggagcaggagctggggcgggtgcaggtgctggcggtcgcatgttgtcacga |
| RR-(PA)₇-F | tgctcccgcaccagccgaggcaggtgaagaatgtgactgt |
| TNK-EA₃K(G₄S)₂-R | ctttgcagcggcctccggtcgcatgttgtcacgaatccagtctaggta |
| RR-EA₃K(G₄S)₂-F | cggaggccgctgcaaagggtggaggaggcagtgggggt |
| TNK-(EA₃K)₃-R | tggcagcggcctccttggctgcagcttccggtcgcatgttgtcacga |
| RR-(EA₃K)₃-F | aggaggccgctgccaaagaggctgcagctaaggaggcaggtgaagaatgtgactgt |

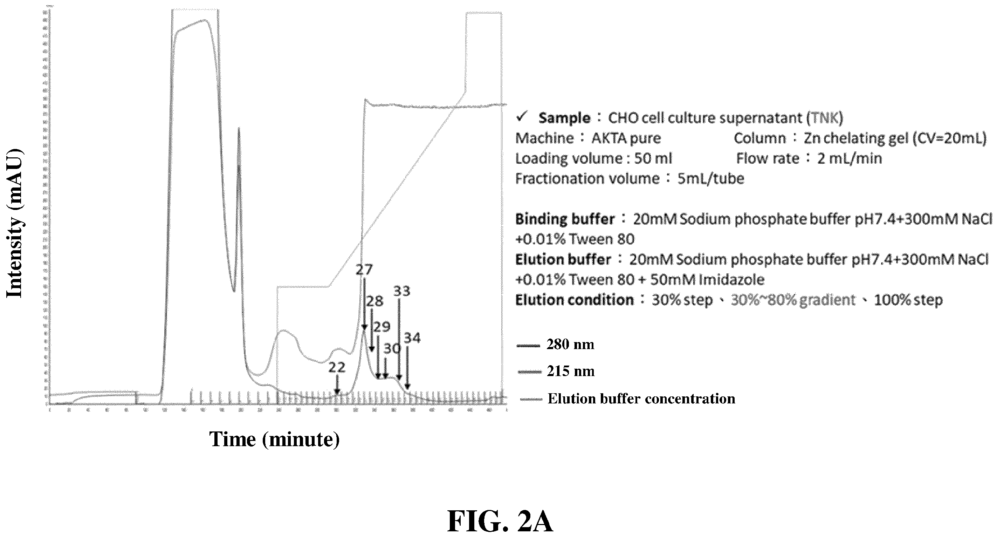

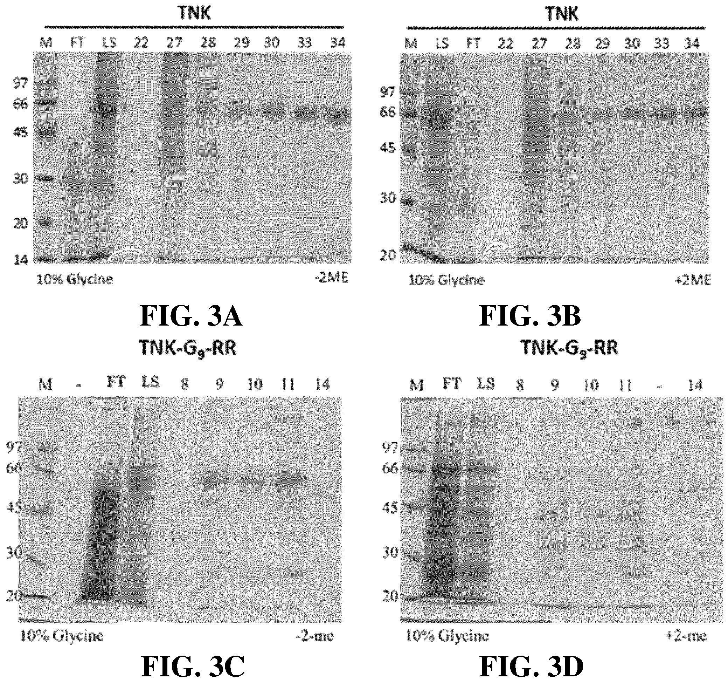

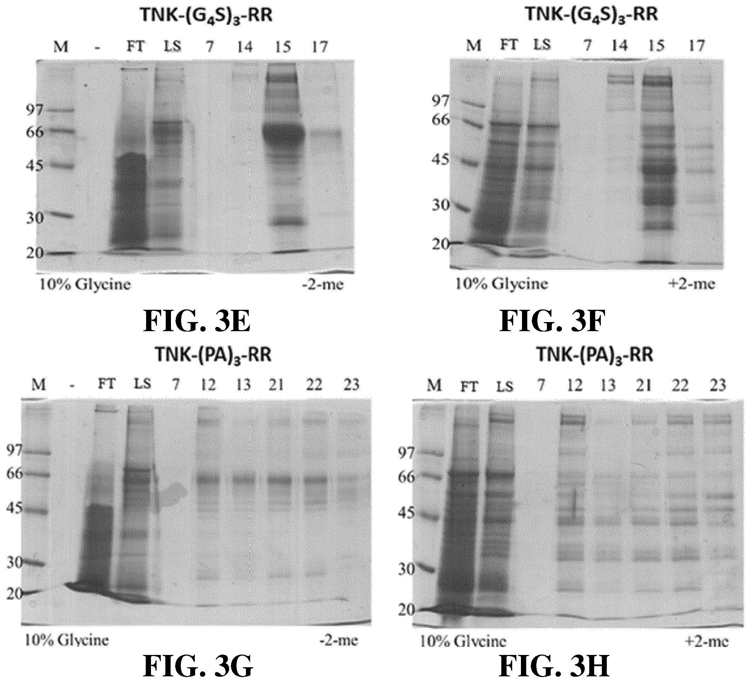

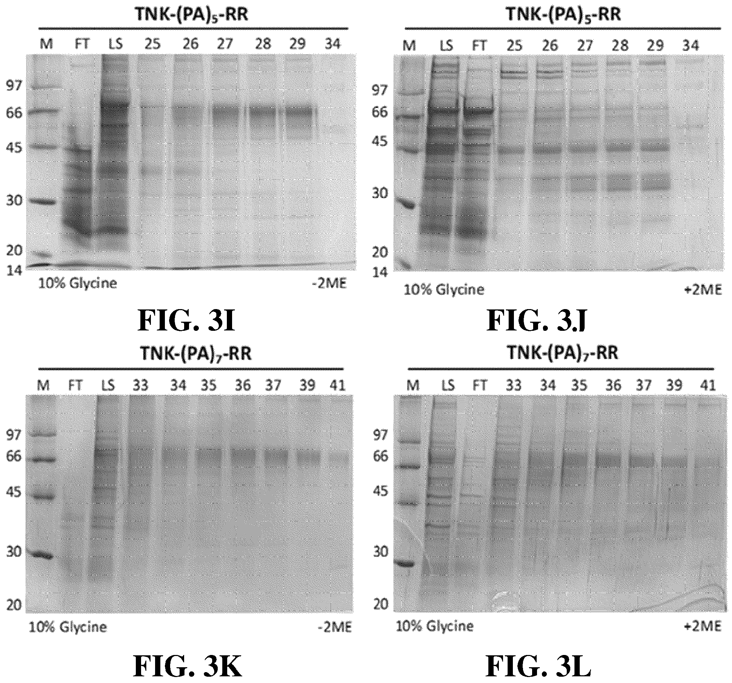

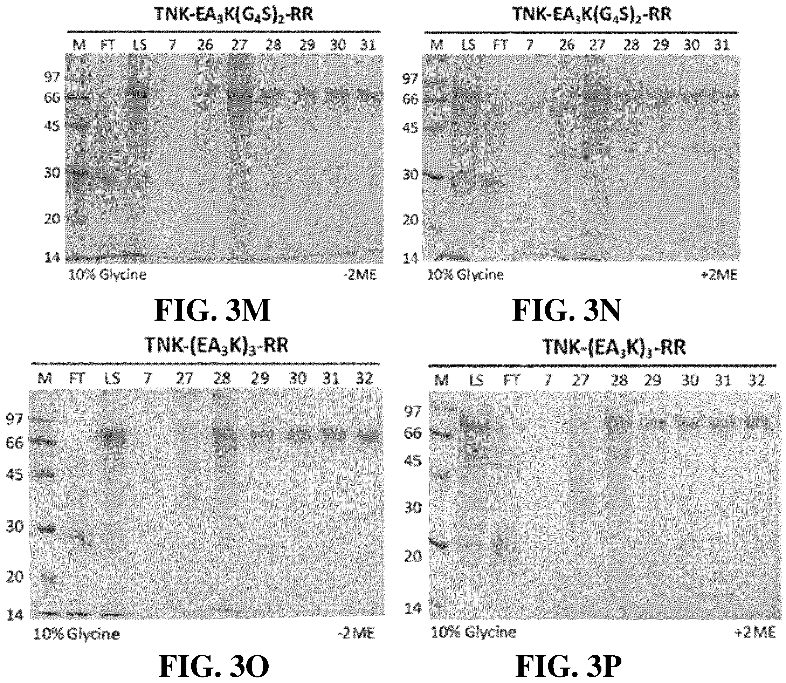

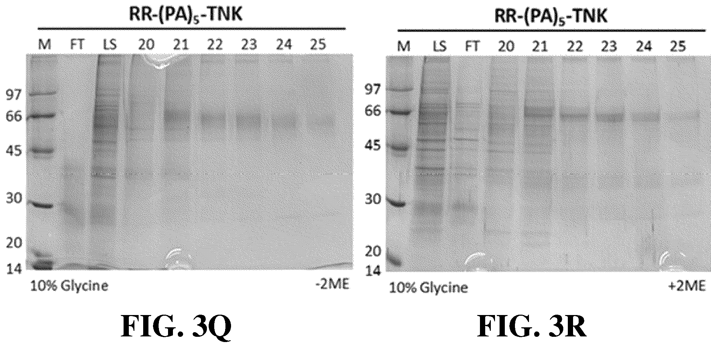

FIG. 2A shows the liquid chromatography result for protein TNK. It is further proven from FIGS. 3A-3B that protein TNK was obtained from fractions 27, 28, 29, 30, 33, and 34.

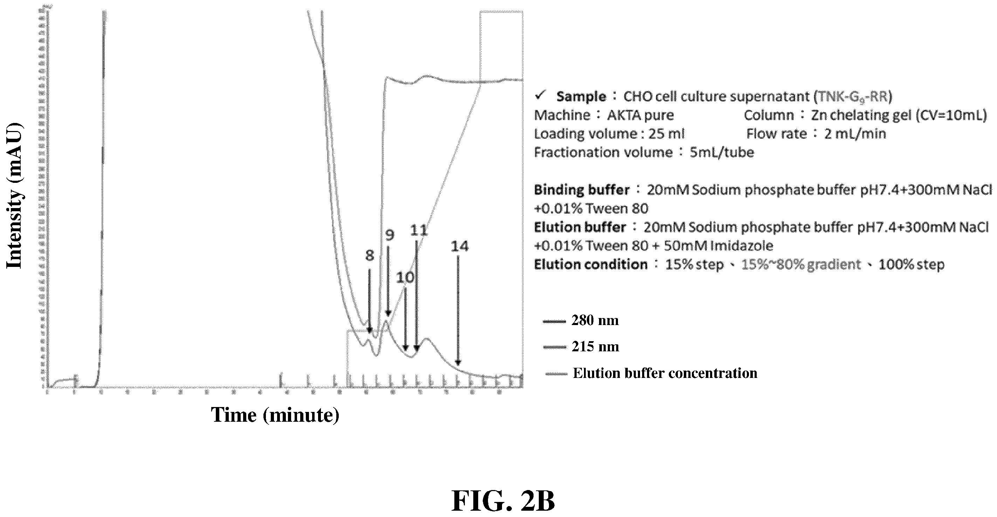

FIG. 2B shows the liquid chromatography result for protein TNK-G₉-RR. It is further proven from FIGS. 3C-3D that protein TNK-G₉-RR was obtained from fractions 9, 10, and 11.

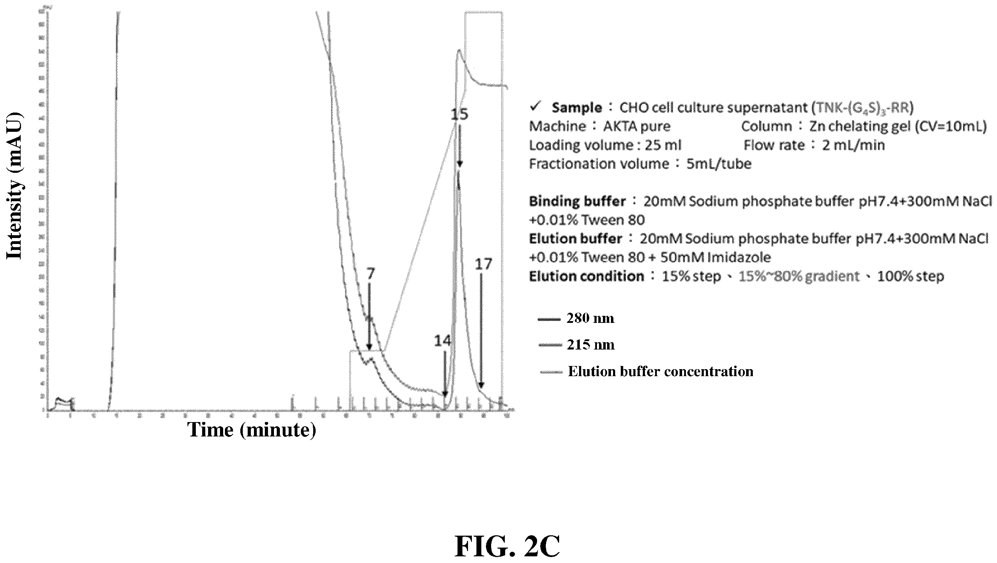

FIG. 2C shows the liquid chromatography result for protein TNK-(G₄S)₃-RR. It is further proven from FIGS. 3E-3F that protein TNK-(G₄S)₃-RR was obtained from fraction 15.

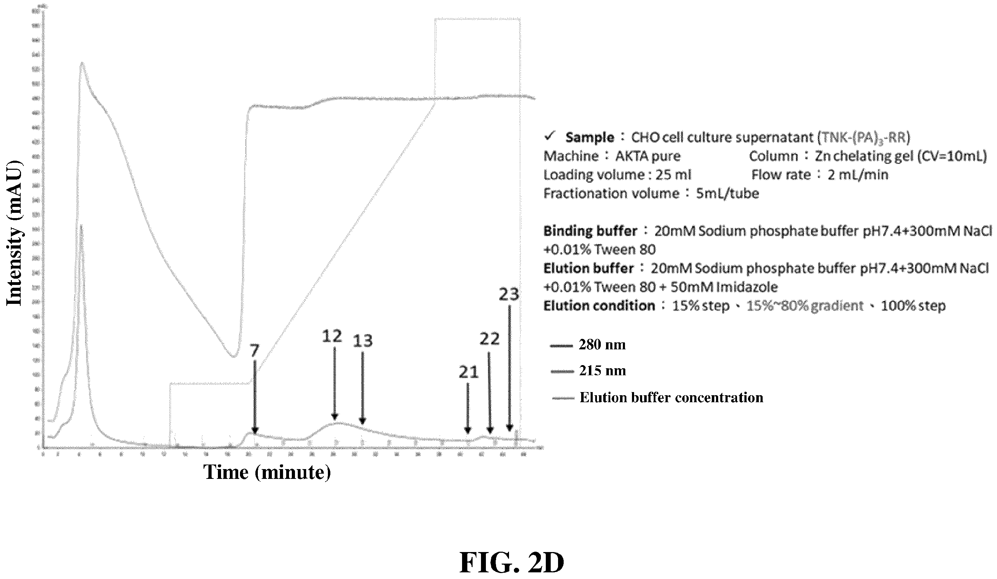

FIG. 2D shows the liquid chromatography result for protein TNK-(PA)₃-RR. It is further proven from FIGS. 3G-3H that protein TNK-(PA)₃-RR was obtained from fractions 12, 13, 21, and 22.

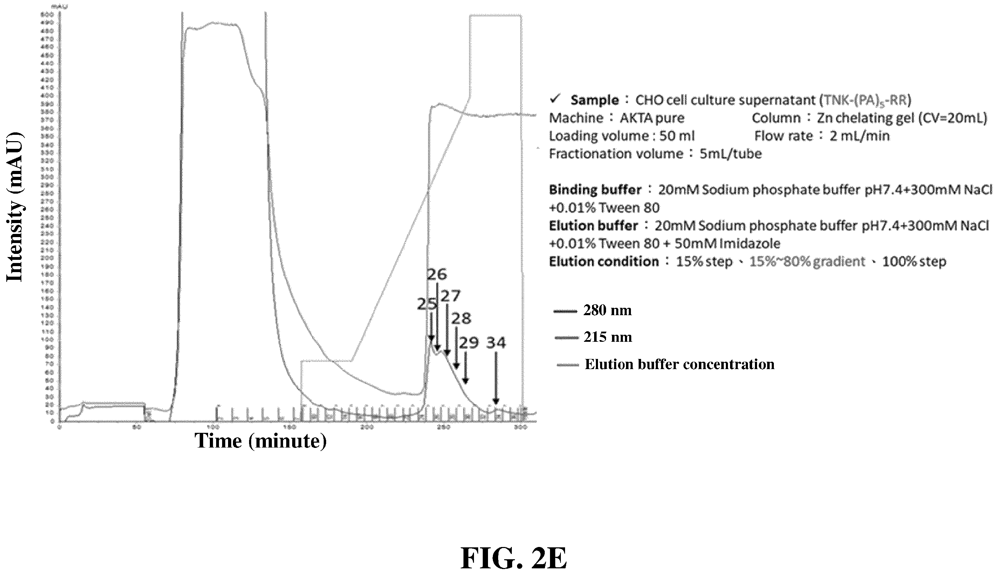

FIG. 2E shows the liquid chromatography result for protein TNK-(PA)₅-RR. It is further proven from FIGS. 3I-3J that protein TNK-(PA)₅-RR was obtained from fractions 27, 28, and 29.

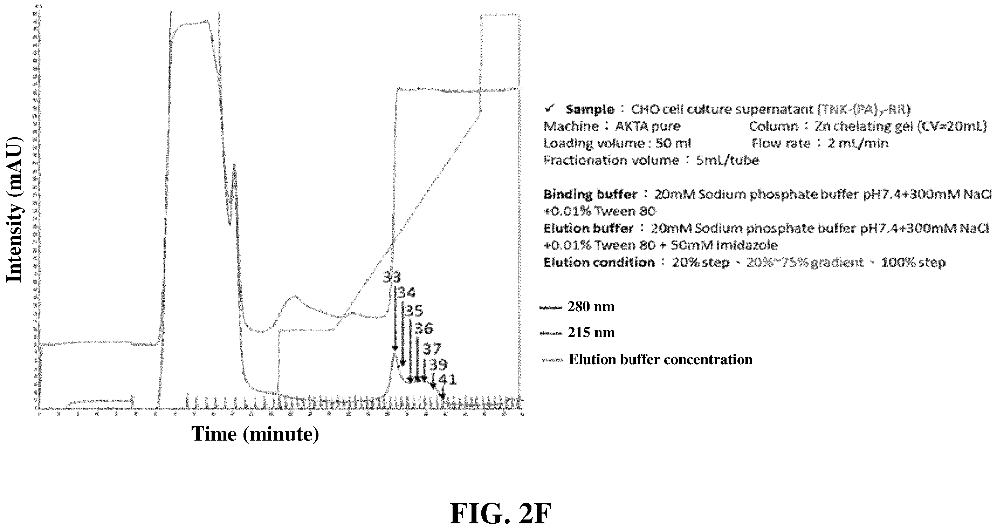

FIG. 2F shows the liquid chromatography result for protein TNK-(PA)₇-RR. It is further proven from FIGS. 3K-3L that protein TNK-(PA)₇-RR was obtained from fractions 33, 34, 35, 36, 37, and 39.

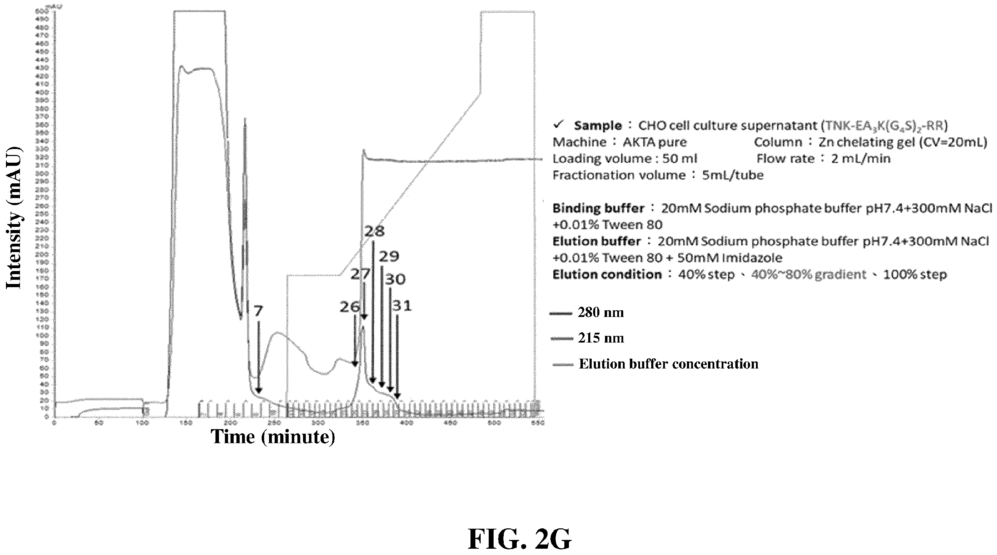

FIG. 2G shows the liquid chromatography result for protein TNK-EA₃K(G₄S)₂-RR. It is further proven from FIGS. 3M-3N that protein TNK-EA₃K(G₄S)₂-RR was obtained from fractions 27, 28, 29, 30, and 31.

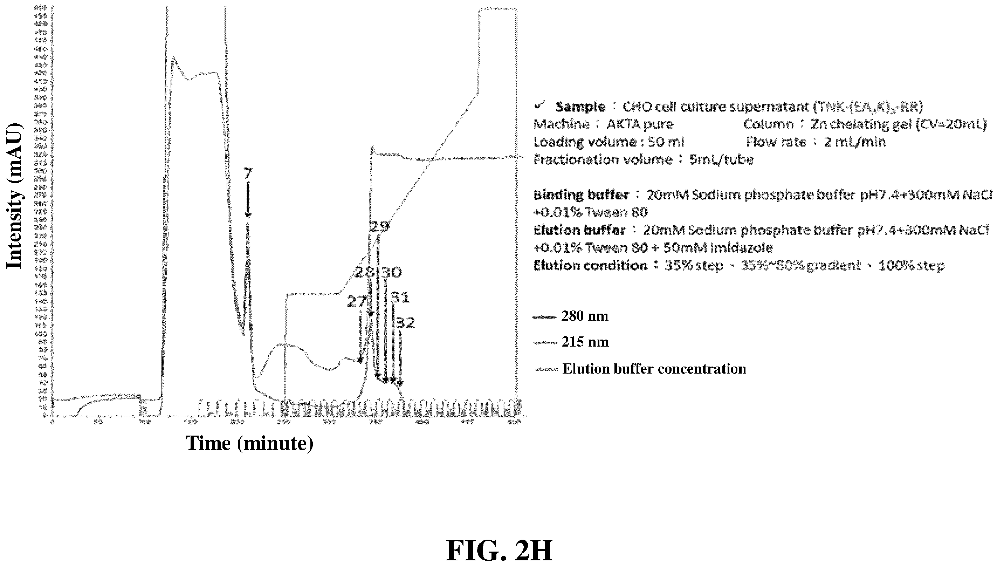

FIG. 2H shows the liquid chromatography result for protein TNK-(EA₃K)₃-RR. It is further proven from FIGS. 3O-3P that protein TNK-(EA₃K)₃-RR was obtained from fractions 28, 29, 30, 31, and 32.

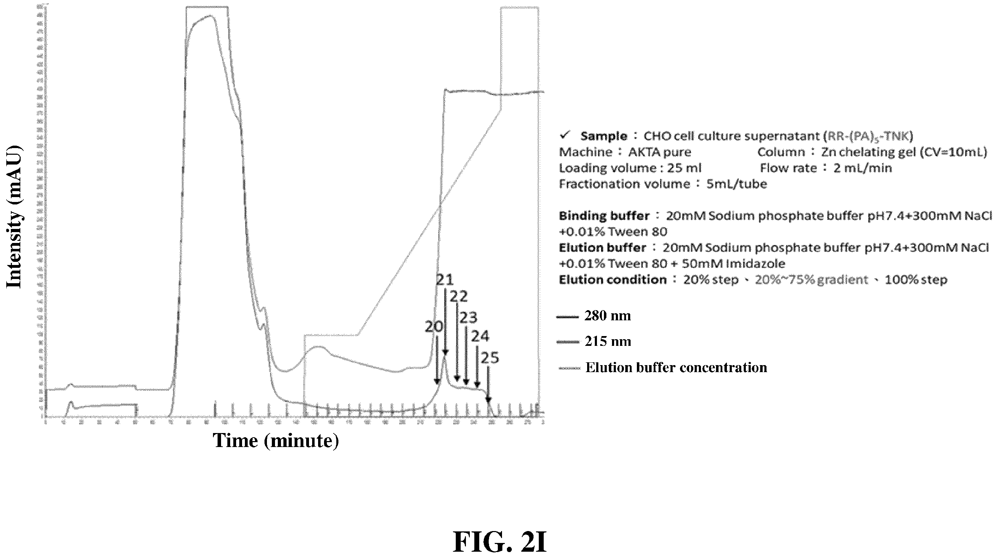

FIG. 2I shows the liquid chromatography result for protein RR-(PA)₃-TNK. It is further proven from FIGS. 3Q-3R that protein RR-(PA)₃-TNK was obtained from fractions 21, 22, 23, and 24.

The amino acid sequences of all proteins are listed in Table 2, and the yield of each protein is listed in Table 3. It is learnt that all protein including protein TNK, protein RR, and fusion proteins containing protein TNK have a certain yield.

**Table 2. Protein sequence**

| Protein Name | Amino acid sequence | SEQ ID No. |
|---|---|---|
| TNK | SYQVICRDEKTQMIYQQHQSWLRPVLRSNRVEYCWCNSGRAQCHSVPVKS CSEPRCFNGGTCQQALYFSDFVCQCPEGFAGKCCEIDTRATCYEDQGISYRG NWSTAESGAECTNWQSSALAQKPYSGRRPDAIRLGLGNHNYCRNPDRDSK PWCYVFKAGKYSSEFCSTPACSEGNSDCYFGNGSAYRGTHSLTESGASCLP WNSMILIGKVYTAQNPSAQALGLGKHNYCRNPDGDAKPWCHVLKNRRLT WEYCDVPSCSTCGLRQYSQPQFRIKGGLFADIASHPWQAAIFAAAAASPGE RFLCGGILISSCWILSAAHCFQERFPPHHLTVILGRTYRVVPGEEEQKFEVEK YIVHKEFDDDTYDNDIALLQLKSDSSRCAQESSVVRTVCLPPADLQLPDWT ECELSGYGKHEALSPFYSERLKEAHVRLYPSSRCTSQHLLNRTVTDNMLCA | 10 |
| | GDTRSGGPQANLHDACQGDSGGPLVCLNDGRMTLVGIISWGLGCGQKDVP GVYTKVTNYLDWIRDNMRP | |
| RR | EAGEECDCGSPENPCCDAATCKLRPGAQCAEGLCCDQCRFKKKRTICRRAK GDRRDDRCTGQSADCPRNRLYG | 34 |
| TNK-G₉-RR | SYQVICRDEKTQMIYQQHQSWLRPVLRSNRVEYCWCNSGRAQCHSVPVKS CSEPRCFNGGTCQQALYFSDFVCQCPEGFAGKCCEIDTRATCYEDQGISYRG NWSTAESGAECTNWQSSALAQKPYSGRRPDAIRLGLGNHNYCRNPDRDSK PWCYVFKAGKYSSEFCSTPACSEGNSDCYFGNGSAYRGTHSLTESGASCLP WNSMILIGKVYTAQNPSAQALGLGKHNYCRNPDGDAKPWCHVLKNRRLT WEYCDVPSCSTCGLRQYSQPQFRIKGGLFADIASHPWQAAIFAAAAASPGE RFLCGGILISSCWILSAAHCFQERFPPHHLTVILGRTYRVVPGEEEQKFEVEK YIVHKEFDDDTYDNDIALLQLKSDSSRCAQESSVVRTVCLPPADLQLPDWT ECELSGYGKHEALSPFYSERLKEAHVRLYPSSRCTSQHLLNRTVTDNMLCA GDTRSGGPQANLHDACQGDSGGPLVCLNDGRMTLVGIISWGLGCGQKDVP GVYTKVTNYLDWIRDNMRPGGGGGGGGEAGEECDCGSPENPCCDAAT CKLRPGAQCAEGLCCDQCRFKKKRTICRRAKGDRRDDRCTGQSADCPRNR LYG | 35 |
| TNK-(G₄S)₃-RR | SYQVICRDEKTQMIYQQHQSWLRPVLRSNRVEYCWCNSGRAQCHSVPVKS CSEPRCFNGGTCQQALYFSDFVCQCPEGFAGKCCEIDTRATCYEDQGISYRG NWSTAESGAECTNWQSSALAQKPYSGRRPDAIRLGLGNHNYCRNPDRDSK PWCYVFKAGKYSSEFCSTPACSEGNSDCYFGNGSAYRGTHSLTESGASCLP WNSMILIGKVYTAQNPSAQALGLGKHNYCRNPDGDAKPWCHVLKNRRLT WEYCDVPSCSTCGLRQYSQPQFRIKGGLFADIASHPWQAAIFAAAAASPGE RFLCGGILISSCWILSAAHCFQERFPPHHLTVILGRTYRVVPGEEEQKFEVEK YIVHKEFDDDTYDNDIALLQLKSDSSRCAQESSVVRTVCLPPADLQLPDWT | 36 |
| | ECELSGYGKHEALSPFYSERLKEAHVRLYPSSRCTSQHLLNRTVTDNMLCA GDTRSGGPQANLHDACQGDSGGPLVCLNDGRMTLVGIISWGLGCGQKDVP GVYTKVTNYLDWIRDNMRPGGGGSGGGGSGGGGSEAGEECDCGSPENP CCDAATCKLRPGAQCAEGLCCDQCRFKKKRTICRRAKGDRRDDRCTGQSA DCPRNRLYG | |
| TNK-(PA)₃-RR | SYQVICRDEKTQMIYQQHQSWLRPVLRSNRVEYCWCNSGRAQCHSVPVKS CSEPRCFNGGTCQQALYFSDFVCQCPEGFAGKCCEIDTRATCYEDQGISYRG NWSTAESGAECTNWQSSALAQKPYSGRRPDAIRLGLGNHNYCRNPDRDSK PWCYVFKAGKYSSEFCSTPACSEGNSDCYFGNGSAYRGTHSLTESGASCLP WNSMILIGKVYTAQNPSAQALGLGKHNYCRNPDGDAKPWCHVLKNRRLT WEYCDVPSCSTCGLRQYSQPQFRIKGGLFADIASHPWQAAIFAAAAASPGE RFLCGGILISSCWILSAAHCFQERFPPHHLTVILGRTYRVVPGEEEQKFEVEK YIVHKEFDDDTYDNDIALLQLKSDSSRCAQESSVVRTVCLPPADLQLPDWT ECELSGYGKHEALSPFYSERLKEAHVRLYPSSRCTSQHLLNRTVTDNMLCA GDTRSGGPQANLHDACQGDSGGPLVCLNDGRMTLVGIISWGLGCGQKDVP GVYTKVTNYLDWIRDNMRPPAPAPAEAGEECDCGSPENPCCDAATCKLRP GAQCAEGLCCDQCRFKKKRTICRRAKGDRRDDRCTGQSADCPRNRLYG | 37 |
| TNK-(PA)₅-RR | SYQVICRDEKTQMIYQQHQSWLRPVLRSNRVEYCWCNSGRAQCHSVPVKS CSEPRCFNGGTCQQALYFSDFVCQCPEGFAGKCCEIDTRATCYEDQGISYRG NWSTAESGAECTNWQSSALAQKPYSGRRPDAIRLGLGNHNYCRNPDRDSK PWCYVFKAGKYSSEFCSTPACSEGNSDCYFGNGSAYRGTHSLTESGASCLP WNSMILIGKVYTAQNPSAQALGLGKHNYCRNPDGDAKPWCHVLKNRRLT WEYCDVPSCSTCGLRQYSQPQFRIKGGLFADIASHPWQAAIFAAAAASPGE RFLCGGILISSCWILSAAHCFQERFPPHHLTVILGRTYRVVPGEEEQKFEVEK YIVHKEFDDDTYDNDIALLQLKSDSSRCAQESSVVRTVCLPPADLQLPDWT | 38 |
| | ECELSGYGKHEALSPFYSERLKEAHVRLYPSSRCTSQHLLNRTVTDNMLCA GDTRSGGPQANLHDACQGDSGGPLVCLNDGRMTLVGIISWGLGCGQKDVP GVYTKVTNYLDWIRDNMRPPAPAPAPAPAEAGEECDCGSPENPCCDAATC KLRPGAQCAEGLCCDQCRFKKKRTICRRAKGDRRDDRCTGQSADCPRNRL YG | |
| TNK-(PA)₇-RR | SYQVICRDEKTQMIYQQHQSWLRPVLRSNRVEYCWCNSGRAQCHSVPVKS CSEPRCFNGGTCQQALYFSDFVCQCPEGFAGKCCEIDTRATCYEDQGISYRG NWSTAESGAECTNWQSSALAQKPYSGRRPDAIRLGLGNHNYCRNPDRDSK PWCYVFKAGKYSSEFCSTPACSEGNSDCYFGNGSAYRGTHSLTESGASCLP WNSMILIGKVYTAQNPSAQALGLGKHNYCRNPDGDAKPWCHVLKNRRLT WEYCDVPSCSTCGLRQYSQPQFRIKGGLFADIASHPWQAAIFAAAAASPGE RFLCGGILISSCWILSAAHCFQERFPPHHLTVILGRTYRVVPGEEEQKFEVEK YIVHKEFDDDTYDNDIALLQLKSDSSRCAQESSVVRTVCLPPADLQLPDWT ECELSGYGKHEALSPFYSERLKEAHVRLYPSSRCTSQHLLNRTVTDNMLCA GDTRSGGPQANLHDACQGDSGGPLVCLNDGRMTLVGIISWGLGCGQKDVP GVYTKVTNYLDWIRDNMRPPAPAPAPAPAPAPAEAGEECDCGSPENPCCD AATCKLRPGAQCAEGLCCDQCRFKKKRTICRRAKGDRRDDRCTGQSADCP RNRLYG | 39 |
| TNK-EA₃K(G₄S )₂-RR | SYQVICRDEKTQMIYQQHQSWLRPVLRSNRVEYCWCNSGRAQCHSVPVKS CSEPRCFNGGTCQQALYFSDFVCQCPEGFAGKCCEIDTRATCYEDQGISYRG NWSTAESGAECTNWQSSALAQKPYSGRRPDAIRLGLGNHNYCRNPDRDSK PWCYVFKAGKYSSEFCSTPACSEGNSDCYFGNGSAYRGTHSLTESGASCLP WNSMILIGKVYTAQNPSAQALGLGKHNYCRNPDGDAKPWCHVLKNRRLT WEYCDVPSCSTCGLRQYSQPQFRIKGGLFADIASHPWQAAIFAAAAASPGE RFLCGGILISSCWILSAAHCFQERFPPHHLTVILGRTYRVVPGEEEQKFEVEK | 40 |
| | YIVHKEFDDDTYDNDIALLQLKSDSSRCAQESSVVRTVCLPPADLQLPDWT ECELSGYGKHEALSPFYSERLKEAHVRLYPSSRCTSQHLLNRTVTDNMLCA GDTRSGGPQANLHDACQGDSGGPLVCLNDGRMTLVGIISWGLGCGQKDVP GVYTKVTNYLDWIRDNMRPEAAAKGGGGSGGGGSEAGEECDCGSPENP CCDAATCKLRPGAQCAEGLCCDQCRFKKKRTICRRAKGDRRDDRCTGQSA DCPRNRLYG | |
| TNK-(EA₃K)₃-RR | SYQVICRDEKTQMIYQQHQSWLRPVLRSNRVEYCWCNSGRAQCHSVPVKS CSEPRCFNGGTCQQALYFSDFVCQCPEGFAGKCCEIDTRATCYEDQGISYRG NWSTAESGAECTNWQSSALAQKPYSGRRPDAIRLGLGNHNYCRNPDRDSK PWCYVFKAGKYSSEFCSTPACSEGNSDCYFGNGSAYRGTHSLTESGASCLP WNSMILIGKVYTAQNPSAQALGLGKHNYCRNPDGDAKPWCHVLKNRRLT WEYCDVPSCSTCGLRQYSQPQFRIKGGLFADIASHPWQAAIFAAAAASPGE RFLCGGILISSCWILSAAHCFQERFPPHHLTVILGRTYRVVPGEEEQKFEVEK YIVHKEFDDDTYDNDIALLQLKSDSSRCAQESSVVRTVCLPPADLQLPDWT ECELSGYGKHEALSPFYSERLKEAHVRLYPSSRCTSQHLLNRTVTDNMLCA GDTRSGGPQANLHDACQGDSGGPLVCLNDGRMTLVGIISWGLGCGQKDVP GVYTKVTNYLDWIRDNMRPEAAAKEAAAKEAAAKEAGEECDCGSPENPC CDAATCKLRPGAQCAEGLCCDQCRFKKKRTICRRAKGDRRDDRCTGQSAD CPRNRLYG | 41 |
| RR-(PA)₅-TNK | EAGEECDCGSPENPCCDAATCKLRPGAQCAEGLCCDQCRFKKKRTICRRAK GDRRDDRCTGQSADCPRNRLYGPAPAPAPAPASYQVICRDEKTQMIYQQH QSWLRPVLRSNRVEYCWCNSGRAQCHSVPVKSCSEPRCFNGGTCQQALYF SDFVCQCPEGFAGKCCEIDTRATCYEDQGISYRGNWSTAESGAECTNWQSS ALAQKPYSGRRPDAIRLGLGNHNYCRNPDRDSKPWCYVFKAGKYSSEFCS TPACSEGNSDCYFGNGSAYRGTHSLTESGASCLPWNSMILIGKVYTAQNPS | 42 |
| | AQALGLGKHNYCRNPDGDAKPWCHVLKNRRLTWEYCDVPSCSTCGLRQY SQPQFRIKGGLFADIASHPWQAAIFAAAAASPGERFLCGGILISSCWILSAAH CFQERFPPHHLTVILGRTYRVVPGEEEQKFEVEKYIVHKEFDDDTYDNDIAL LQLKSDSSRCAQESSVVRTVCLPPADLQLPDWTECELSGYGKHEALSPFYSE RLKEAHVRLYPSSRCTSQHLLNRTVTDNMLCAGDTRSGGPQANLHDACQG DSGGPLVCLNDGRMTLVGIISWGLGCGQKDVPGVYTKVTNYLDWIRDNM RP | |

A linker sequence is within a box

**Table 3. Protein yield**

| Protein | Expression system | Purification by liquid chromatography | Yield (mg/L) |
|---|---|---|---|
| TNK | CHO cell | Zinc chelating resin | 88 |
| RR | Yeast cell | Cation exchange resin | 6 |
| TNK-G₉-RR | CHO cell | Zinc chelating resin | 62 |
| TNK-(G₄S)₃-RR | CHO cell | Zinc chelating resin | 65 |
| TNK-(PA)₃-RR | CHO cell | Zinc chelating resin | 60 |
| TNK-(PA)₅-RR | CHO cell | Zinc chelating resin | 70 |
| TNK-(PA)₇-RR | CHO cell | Zinc chelating resin | 44 |
| TNK-EA₃K(G₄S)₂-RR | CHO cell | Zinc chelating resin | 24 |
| TNK-(EA₃K)₃-RR | CHO cell | Zinc chelating resin | 21 |
| RR-(PA)₃-TNK | CHO cell | Zinc chelating resin | 14 |

### Example 2: Thrombolysis Test

Whole blood thrombolytic plate assay was performed. Blood was collected from healthy volunteers and then was mixed with 3.8% tri-sodium citrate at a ratio 9: 1. A clotting mixture was prepared through mixing thrombin (6.25x10⁻³ U) and calcium chloride (250 mM) in a HEPES buffer (25 mM HEPES, 137 mM NaCl). Five µL of the clotting mixture was deposited onto the bottom edge of the well of a 96-well microplate, followed by addition of 25 µL of the blood mixture. The plate was then sealed and incubated at 37□ for 30 minutes, and blood clots were formed around the edge of the well.

Each protein was diluted with a HEPES solution for different final concentrations in total volume of 70 µL. Each protein sample was added into the well containing blood clots and reacted with the blood clots at 37□ for 120 minutes in an ELISA reader. During the reaction, each ELISA plate was shaken once every minute (200 rpm) and the absorbance at 510 nm was measured once every three minutes using the ELISA reader. Since the dissolved blood clots could flow to cover the center of the well during the reaction, the absorbance at 510 nm could be used to determine time to 50% thrombus lysis (T0.5, minute). As such, the thrombolysis activity could be obtained.

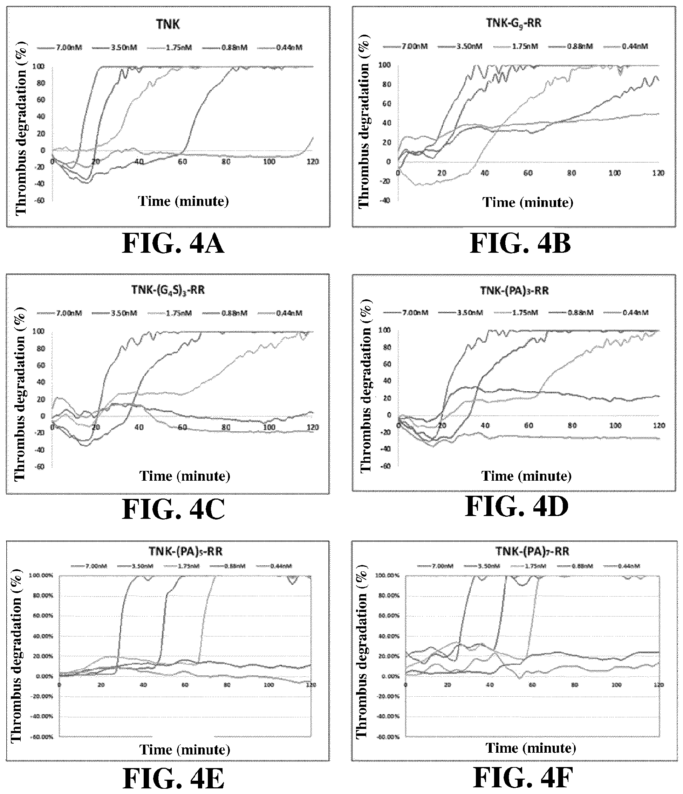

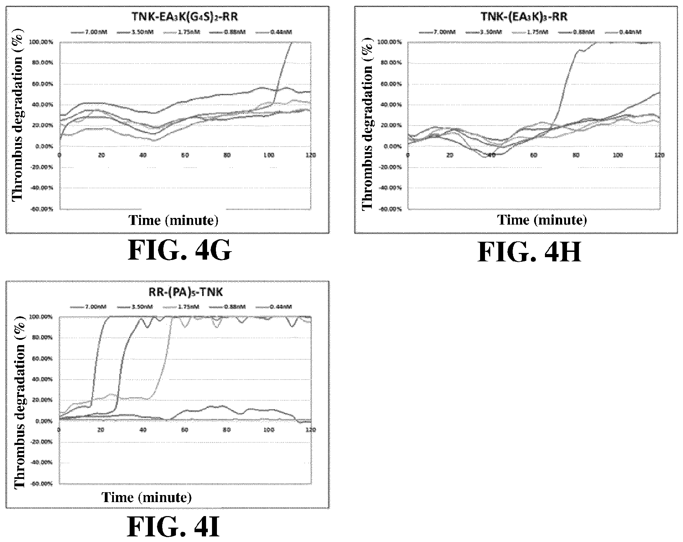

The thrombolysis activities of all proteins at different concentrations are presented in FIGS. 4A-4I. Additionally, the time to 50% thrombus lysis of each protein at different concentrations is presented in Table 4.

**Table 4. Time to 50% thrombus lysis of each protein at different concentrations**

| Protein | T0.5: time to 50% thrombus lysis (minute) | | | | |
|---|---|---|---|---|---|
| | 7.0 nM | 3.5 nM | 1.75 nM | 0.88 nM | 0.44 nM |
| TNK | 19.8±3.2 | 32.3±9.0 | 56.1±17.3 | 70.7±12.4 | ND |
| TNK-G₉-RR | 25.9±2.4 | 35.3±4.5 | 56.7±6.3 | ND | ND |
| TNK-(G₄S)₃-RR | 26.3±2.4 | 37.1±3.3 | 59.1±6.2 | ND | ND |
| TNK-(PA)₃-RR | 25.3±1.6 | 34.2±1.7 | 53.2±1.7 | ND | ND |
| TNK-(PA)₃-RR | 37.4±14.0 | 60.9±19.9 | 85.0±20.9 | ND | ND |
| TNK-(PA)₇-RR | 36.5±14.1 | 54.6±19.3 | 78.9±22.4 | ND | ND |
| TNK-EA₃K(G₄S)₂-RR | 93.0±9.5 | 106.4±22.1 | ND | ND | ND |
| TNK-(EA₃K)₃-RR | 73.9±5.4 | 108.4±20.3 | ND | ND | ND |
| RR-(PA)₃-TNK | 32.7±14.1 | 49.8±20.8 | 75.3±23.7 | ND | ND |

| | | | | | |
|---|---|---|---|---|---|
| ND indicates thrombolysis does not occur within 120 minutes | | | | | |

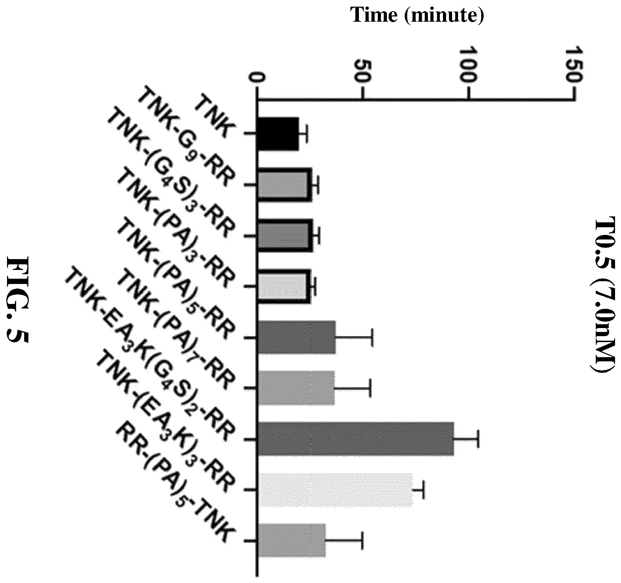

The time to 50% thrombus lysis of each protein at a concentration of 7.0 nM is presented in FIG. 5 and Table 5. It is learnt that the well-known thrombolytic agent TNK and all fusion protein each containing TNK can dissolve blood clots.

**Table 5. Time to 50% thrombus lysis at 7.0 nM**

| Protein | T0.5 (time to 50% thrombus lysis) at 7.0 nM (minute) | Ratio (TNK/corresponding protein) |
|---|---|---|
| TNK | 19.8±3.2 | 1/1 |
| TNK-G₉-RR | 25.9±2.4 | 1/1.31 |
| TNK-(G₄S)₃-RR | 26.3±2.4 | 1/1.33 |
| TNK-(PA)₃-RR | 25.3±1.6 | 1/1.28 |
| TNK-(PA)₃-RR | 37.4±14.0 | 1/1.89 |
| TNK-(PA)₇-RR | 36.5±14.1 | 1/1.84 |
| TNK-EA₃K(G₄S)₂-RR | 93.0±9.5 | 1/4.70 |
| TNK-(EA₃K)₃-RR | 73.9±5.4 | 1/3.73 |
| RR-(PA)₅-TNK | 32.7±14.1 | 1/1.65 |

### Example 3: Inhibition Test Against Platelet Aggregation

Ten mL of venous blood was collected from healthy donors not received any medication for at least two weeks, and then mixed with 3.13% sodium citrate (pH7.4) at a ratio of 9: 1. The blood sample was centrifugated at 1,000 rpm for 10 minutes and the supernatant was obtained to collect platelet-rich plasma (PRP). The remaining part of the blood sample was centrifugated at 4,000 rpm for 10 minutes and the supernatant was obtained to collect platelet-poor plasma (PPP). Meanwhile, all proteins were dissolved in a R+E buffer (2.5 mM Tris, 1.5 mM sodium chloride, 50 mM Arginine, and 50 mM Glutamate) for different final concentrations. One hundred and ninety µL of PRP was mixed with 10 µL of a PBS buffer or 10 µL of the protein solution, and reacted with the corresponding reactant at 37 □ for 1 minute in a platelet aggregometer (Nikoh Bioscience). Ten µL of 200 µM adenosine diphosphate was added to induce platelet aggregation and the response of platelet aggregation was monitored by light transmission. The result was expressed as the average of a ratio relative to inhibitory activity of the control group.

The inhibitory activities of different proteins against platelet aggregation are presented in Table 6. Relative to the well-known antiplatelet agent trimucrin variant RR, each protein has a lower inhibitory activity against platelet aggregation.

**Table 6. IC₅₀ for platelet aggregation**

| Protein | IC₅₀: platelet aggregation (nM) | Ratio (RR/corresponding protein) |
|---|---|---|
| RR | 52.5±11.0 | 1/1 |
| TNK-G₉-RR | 567.2±26.8 | 1/10.80 |
| TNK-(G₄S)₃-RR | 249.3±57.5 | 1/4.75 |
| TNK-(PA)₃-RR | 505.5±49.1 | 1/9.63 |
| TNK-(PA)₅-RR | 379.9±31.9 | 1/7.24 |
| TNK-(PA)₇-RR | 347.4±33.6 | 1/6.62 |
| TNK-EA₃K(G₄S)₂-RR | 275.5±67.1 | 1/5.25 |
| TNK-(EA₃K)₃-RR | 457.5±25.7 | 1/8.71 |
| RR-(PA)₅-TNK | 1370.7±155.6 | 1/26.11 |

As described above, it is confirmed that the fusion protein of the present invention can reduce a thrombus, resulting in a low bleeding risk. Therefore, it has a potential to be a candidate thrombolytic agent.

While the invention has been described in connection with what is considered the most practical and preferred embodiments, it is understood that this invention is not limited to the disclosed embodiments but is intended to cover various arrangements included within the spirit and scope of the broadest interpretation so as to encompass all such modifications and equivalent arrangements.

Sequence Listing Information:
DTD Version: V1_3
File Name: PI-111-157-PCT.xml
Software Name: WIPO Sequence
Software Version: 2.1.1
Production Date: 2022-09-20
General Information:
Current application / IP Office: WO
Current application / Applicant file reference: PI-111-157-PCT
Applicant name: WOEI-JER CHUANG
Applicant name / Language: zh
Applicant name / Name Latin: WOEI-JER CHUANG
Inventor name: WOEI-JER CHUANG
Inventor name / Language: zh
Inventor name / Name Latin: WOEI-JER CHUANG
Invention title: FUSION PROTEIN TARGETING INTERGRIN ALPHA(IIB)BETA3 AND
CONTAINING TISSUE PLASMINOGEN ACTIVATOR OR VARIANT THEREOF AND USE THEREOF ( zh )
Sequence Total Quantity: 42
Sequences:
Sequence Number (ID): 1
Length: 9
Molecule Type: AA
Features Location/Qualifiers:
- source, 1..9
> mol_type, protein
> organism, synthetic construct
Residues:
GGGGGGGGG 9

Sequence Number (ID): 2
Length: 15
Molecule Type: AA
Features Location/Qualifiers:
- source, 1..15
> mol_type, protein
> organism, synthetic construct
Residues:
GGGGSGGGGS GGGGS 15

Sequence Number (ID): 3
Length: 6
Molecule Type: AA
Features Location/Qualifiers:
- source, 1..6
> mol_type, protein
> organism, synthetic construct
Residues:
PAPAPA 6

Sequence Number (ID): 4
Length: 10
Molecule Type: AA
Features Location/Qualifiers:
- source, 1..10
> mol_type, protein
> organism, synthetic construct
Residues:
PAPAPAPAPA 10

Sequence Number (ID): 5
Length: 14
Molecule Type: AA
Features Location/Qualifiers:

- source, 1..14
> mol_type, protein
> organism, synthetic construct
Residues:
PAPAPAPAPA PAPA 14

Sequence Number (ID): 6
Length: 15
Molecule Type: AA
Features Location/Qualifiers:
- source, 1..15
> mol_type, protein
> organism, synthetic construct
Residues:
EAAAKGGGGS GGGGS 15

Sequence Number (ID): 7
Length: 15
Molecule Type: AA
Features Location/Qualifiers:
- source, 1..15
> mol_type, protein
> organism, synthetic construct
Residues:
EAAAKEAAAK EAAAK 15

Sequence Number (ID): 8
Length: 527
Molecule Type: AA
Features Location/Qualifiers:
- source, 1..527
> mol_type, protein
> organism, synthetic construct
Residues:
SYQVICRDEK TQMIYQQHQS WLRPVLRSNR VEYCWCNSGR AQCHSVPVKS CSEPRCFNGG 60
TCQQALYFSD FVCQCPEGFA GKCCEIDTRA TCYEDQGISY RGTWSTAESG AECTNWNSSA 120
LAQKPYSGRR PDAIRLGLGN HNYCRNPDRD SKPWCYVFKA GKYSSEFCST PACSEGNSDC 180
YFGNGSAYRG THSLTESGAS CLPWNSMILI GKVYTAQNPS AQALGLGKHN YCRNPDGDAK 240
PWCHVLKNRR LTWEYCDVPS CSTCGLRQYS QPQFRIKGGL FADIASHPWQ AAIFAKHRRS 300
PGERFLCGGI LISSCWILSA AHCFQERFPP HHLTVILGRT YRVVPGEEEQ KFEVEKYIVH 360
KEFDDDTYDN DIALLQLKSD SSRCAQESSV VRTVCLPPAD LQLPDWTECE LSGYGKHEAL 420
SPFYSERLKE AHVRLYPSSR CTSQHLLNRT VTDNMLCAGD TRSGGPQANL HDACQGDSGG 480
PLVCLNDGRM TLVGIISWGL GCGQKDVPGV YTKVTNYLDW IRDNMRP 527

Sequence Number (ID): 9
Length: 355
Molecule Type: AA
Features Location/Qualifiers:
- source, 1..355
> mol_type, protein
> organism, synthetic construct
Residues:
SYQGNSDCYF GNGSAYRGTH SLTESGASCL PWNSMILIGK VYTAQNPSAQ ALGLGKHNYC 60
RNPDGDAKPW CHVLKNRRLT WEYCDVPSCS TCGLRQYSQP QFRIKGGLFA DIASHPWQAA 120
IFAKHRRSPG ERFLCGGILI SSCWILSAAH CFQERFPPHH LTVILGRTYR VVPGEEEQKF 180
EVEKYIVHKE FDDDTYDNDI ALLQLKSDSS RCAQESSVVR TVCLPPADLQ LPDWTECELS 240
GYGKHEALSP FYSERLKEAH VRLYPSSRCT SQHLLNRTVT DNMLCAGDTR SGGPQANLHD 300
ACQGDSGGPL VCLNDGRMTL VGIISWGLGC GQKDVPGVYT KVTNYLDWIR DNMRP 355

Sequence Number (ID): 10
Length: 527
Molccule Typc: AA
Features Location/Qualifiers:

- source, 1..527
        > mol_type, protein
        > organism, synthetic construct
Residues:
SYQVICRDEK  TQMIYQQHQS  WLRPVLRSNR  VEYCWCNSGR  AQCHSVPVKS  CSEPRCFNGG  60
TCQQALYFSD  FVCQCPEGFA  GKCCEIDTRA  TCYEDQGISY  RGNWSTAESG  AECTNWQSSA  120
LAQKPYSGRR  PDAIRLGLGN  HNYCRNPDRD  SKPWCYVFKA  GKYSSEFCST  PACSEGNSDC  180
YFGNGSAYRG  THSLTESGAS  CLPWNSMILI  GKVYTAQNPS  AQALGLGKHN  YCRNPDGDAK  240
PWCHVLKNRR  LTWEYCDVPS  CSTCGLRQYS  QPQFRIKGGL  FADIASHPWQ  AAIFAAAAAS  300
PGERFLCGGI  LISSCWILSA  AHCFQERFPP  HHLTVILGRT  YRVVPGEEEQ  KFEVEKYIVH  360
KEFDDDTYDN  DIALLQLKSD  SSRCAQESSV  VRTVCLPPAD  LQLPDWTECE  LSGYGKHEAL  420
SPFYSERLKE  AHVRLYPSSR  CTSQHLLNRT  VTDNMLCAGD  TRSGGPQANL  HDACQGDSGG  480
PLVCLNDGRM  TLVGIISWGL  GCGQKDVPGV  YTKVTNYLDW  IRDNMRP  527

Sequence Number (ID): 11
Length: 5
Molecule Type: AA
Features Location/Qualifiers:
        - source, 1..5
        > mol_type, protein
        > organism, Protobothrops mucrosquamatus
Residues:
KKKRT  5

Sequence Number (ID): 12
Length: 5
Molecule Type: AA
Features Location/Qualifiers:
        - source, 1..5
        > mol_type, protein
        > organism, Calloselasma rhodostoma
Residues:
SRAGK  5

Sequence Number (ID): 13
Length: 5
Molecule Type: AA
Features Location/Qualifiers:
        - source, 1..5
        > mol_type, protein
        > organism, synthetic construct
Residues:
IEEGT  5

Sequence Number (ID): 14
Length: 5
Molecule Type: AA
Features Location/Qualifiers:
        - source, 1..5
        > mol_type, protein
        > organism, synthetic construct
Residues:
KKART  5

Sequence Number (ID): 15
Length: 5
Molecule Type: AA
Features Location/Qualifiers:
        - source, 1..5
        > mol_type, protein
        > organism, synthetic construct
Residues:
MKKGT  5

Sequence Number (ID): 16
Length: 6
Molecule Type: AA
Features Location/Qualifiers:
- source, 1..6
> mol_type, protein
> organism, synthetic construct
Residues:
AKGDHP 6

Sequence Number (ID): 17
Length: 6
Molecule Type: AA
Features Location/Qualifiers:
- source, 1..6
> mol_type, protein
> organism, synthetic construct
Residues:
AKGDYP 6

Sequence Number (ID): 18
Length: 6
Molecule Type: AA
Features Location/Qualifiers:
- source, 1..6
> mol_type, protein
> organism, synthetic construct
Residues:
AKGDWP 6

Sequence Number (ID): 19
Length: 6
Molecule Type: AA
Features Location/Qualifiers:
- source, 1..6
> mol_type, protein
> organism, synthetic construct
Residues:
AKGDRP 6

Sequence Number (ID): 20
Length: 6
Molecule Type: AA
Features Location/Qualifiers:
- source, 1..6
> mol_type, protein
> organism, synthetic construct
Residues:
AKGDRR 6

Sequence Number (ID): 21
Length: 6
Molecule Type: AA
Features Location/Qualifiers:
- source, 1..6
> mol_type, protein
> organism, synthetic construct
Residues:
AKGDRK 6

Sequence Number (ID): 22
Length: 6

Molecule Type: AA
Features Location/Qualifiers:
        - source, 1..6
                > mol_type, protein
                > organism, synthetic construct
Residues:
AKGDKR                                                                          6

Sequence Number (ID): 23
Length: 6
Molecule Type: AA
Features Location/Qualifiers:
        - source, 1..6
                > mol_type, protein
                > organism, synthetic construct
Residues:
AKGDKK                                                                          6

Sequence Number (ID): 24
Length: 6
Molecule Type: AA
Features Location/Qualifiers:
        - source, 1..6
                > mol_type, protein
                > organism, synthetic construct
Residues:
AKGDNP                                                                          6

Sequence Number (ID): 25
Length: 6
Molecule Type: AA
Fcatures Location/Qualificrs:
        - source, 1..6
                > mol_type, protein
                > organism, synthetic construct
Residues:
AKGDFP                                                                          6

Sequence Number (ID): 26
Length: 6
Molecule Type: AA
Features Location/Qualifiers:
        - source, 1..6
                > mol_type, protein
                > organism, synthetic construct
Residues:
AKGDWN                                                                          6

Sequence Number (ID): 27
Length: 6
Molecule Type: AA
Features Location/Qualifiers:
        - source, 1..6
                > mol_type, protein
                > organism, synthetic construct
Residues:
PKGDMP                                                                          6

Sequence Number (ID): 28
Length: 6
Molecule Type: AA
Features Location/Qualifiers:
        - source, 1..6

> mol_type, protein
> organism, synthetic construct
Residues:
ARGDWN 6

Sequence Number (ID): 29
Length: 7
Molecule Type: AA
Features Location/Qualifiers:
- source, 1..7
> mol_type, protein
> organism, Protobothrops mucrosquamatus
Residues:
PRNGLYG 7

Sequence Number (ID): 30
Length: 4
Molecule Type: AA
Features Location/Qualifiers:
- source, 1..4
> mol_type, protein
> organism, Calloselasma rhodostoma
Residues:
PRYH 4

Sequence Number (ID): 31
Length: 7
Molecule Type: AA
Features Location/Qualifiers:
- source, 1..7
> mol_type, protein
> organism, synthetic construct
Residues:
PRNRLYG 7

Sequence Number (ID): 32
Length: 4
Molecule Type: AA
Features Location/Qualifiers:
- source, 1..4
> mol_type, protein
> organism, synthetic construct
Residues:
PRNS 4

Sequence Number (ID): 33
Length: 6
Molecule Type: AA
Features Location/Qualifiers:
- source, 1..6
> mol_type, protein
> organism, synthetic construct
Residues:
PRNRFH 6

Sequence Number (ID): 34
Length: 73
Molecule Type: AA
Features Location/Qualifiers:
- source, 1..73
> mol_type, protein
> organism, synthetic construct
Residues:

EAGEECDCGS   PENPCCDAAT   CKLRPGAQCA   EGLCCDQCRF   KKKRTICRRA   KGDRRDDRCT   60
GQSADCPRNR   LYG                                                                73

Sequence Number (ID): 35
Length: 609
Molecule Type: AA
Features Location/Qualifiers:
        - source, 1..609
                > mol_type, protein
                > organism, synthetic construct
Residues:
SYQVICRDEK   TQMIYQQHQS   WLRPVLRSNR   VEYCWCNSGR   AQCHSVPVKS   CSEPRCFNGG   60
TCQQALYFSD   FVCQCPEGFA   GKCCEIDTRA   TCYEDQGISY   RGNWSTAESG   AECTNWQSSA   120
LAQKPYSGRR   PDAIRLGLGN   HNYCRNPDRD   SKPWCYVFKA   GKYSSEFCST   PACSEGNSDC   180
YFGNGSAYRG   THSLTESGAS   CLPWNSMILI   GKVYTAQNPS   AQALGLGKHN   YCRNPDGDAK   240
PWCHVLKNRR   LTWEYCDVPS   CSTCGLRQYS   QPQFRIKGGL   FADIASHPWQ   AAIFAAAAAS   300
PGERFLCGGI   LISSCWILSA   AHCFQERFPP   HHLTVILGRT   YRVVPGEEEQ   KFEVEKYIVH   360
KEFDDDTYDN   DIALLQLKSD   SSRCAQESSV   VRTVCLPPAD   LQLPDWTECE   LSGYGKHEAL   420
SPFYSERLKE   AHVRLYPSSR   CTSQHLLNRT   VTDNMLCAGD   TRSGGPQANL   HDACQGDSGG   480
PLVCLNDGRM   TLVGIISWGL   GCGQKDVPGV   YTKVTNYLDW   IRDNMRPGGG   GGGGGGEAGE   540
ECDCGSPENP   CCDAATCKLR   PGAQCAEGLC   CDQCRFKKKR   TICRRAKGDR   RDDRCTGQSA   600
DCPRNRLYG                                                                       609

Sequence Number (ID): 36
Length: 615
Molecule Type: AA
Features Location/Qualifiers:
        - source, 1..615
                > mol_type, protein
                > organism, synthetic construct
Residues:
SYQVICRDEK   TQMIYQQHQS   WLRPVLRSNR   VEYCWCNSGR   AQCHSVPVKS   CSEPRCFNGG   60
TCQQALYFSD   FVCQCPEGFA   GKCCEIDTRA   TCYEDQGISY   RGNWSTAESG   AECTNWQSSA   120
LAQKPYSGRR   PDAIRLGLGN   HNYCRNPDRD   SKPWCYVFKA   GKYSSEFCST   PACSEGNSDC   180
YFGNGSAYRG   THSLTESGAS   CLPWNSMILI   GKVYTAQNPS   AQALGLGKHN   YCRNPDGDAK   240
PWCHVLKNRR   LTWEYCDVPS   CSTCGLRQYS   QPQFRIKGGL   FADIASHPWQ   AAIFAAAAAS   300
PGERFLCGGI   LISSCWILSA   AHCFQERFPP   HHLTVILGRT   YRVVPGEEEQ   KFEVEKYIVH   360
KEFDDDTYDN   DIALLQLKSD   SSRCAQESSV   VRTVCLPPAD   LQLPDWTECE   LSGYGKHEAL   420
SPFYSERLKE   AHVRLYPSSR   CTSQHLLNRT   VTDNMLCAGD   TRSGGPQANL   HDACQGDSGG   480
PLVCLNDGRM   TLVGIISWGL   GCGQKDVPGV   YTKVTNYLDW   IRDNMRPGGG   GSGGGGSGGG   540
GSEAGEECDC   GSPENPCCDA   ATCKLRPGAQ   CAEGLCCDQC   RFKKKRTICR   RAKGDRRDDR   600
CTGQSADCPR   NRLYG                                                              615

Sequence Number (ID): 37
Length: 606
Molecule Type: AA
Features Location/Qualifiers:
        - source, 1..606
                > mol_type, protein
                > organism, synthetic construct
Residues:
SYQVICRDEK   TQMIYQQHQS   WLRPVLRSNR   VEYCWCNSGR   AQCHSVPVKS   CSEPRCFNGG   60
TCQQALYFSD   FVCQCPEGFA   GKCCEIDTRA   TCYEDQGISY   RGNWSTAESG   AECTNWQSSA   120
LAQKPYSGRR   PDAIRLGLGN   HNYCRNPDRD   SKPWCYVFKA   GKYSSEFCST   PACSEGNSDC   180
YFGNGSAYRG   THSLTESGAS   CLPWNSMILI   GKVYTAQNPS   AQALGLGKHN   YCRNPDGDAK   240
PWCHVLKNRR   LTWEYCDVPS   CSTCGLRQYS   QPQFRIKGGL   FADIASHPWQ   AAIFAAAAAS   300
PGERFLCGGI   LISSCWILSA   AHCFQERFPP   HHLTVILGRT   YRVVPGEEEQ   KFEVEKYIVH   360
KEFDDDTYDN   DIALLQLKSD   SSRCAQESSV   VRTVCLPPAD   LQLPDWTECE   LSGYGKHEAL   420
SPFYSERLKE   AHVRLYPSSR   CTSQHLLNRT   VTDNMLCAGD   TRSGGPQANL   HDACQGDSGG   480
PLVCLNDGRM   TLVGIISWGL   GCGQKDVPGV   YTKVTNYLDW   IRDNMRPPAP   APAEAGEECD   540
CGSPENPCCD   AATCKLRPGA   QCAEGLCCDQ   CRFKKKRTIC   RRAKGDRRDD   RCTGQSADCP   600
RNRLYG                                                                          606

Sequence Number (ID): 38
Length: 610
Molecule Type: AA
Features Location/Qualifiers:
 - source, 1..610
 > mol_type, protein
 > organism, synthetic construct
Residues:
SYQVICRDEK TQMIYQQHQS WLRPVLRSNR VEYCWCNSGR AQCHSVPVKS CSEPRCFNGG 60
TCQQALYFSD FVCQCPEGFA GKCCEIDTRA TCYEDQGISY RGNWSTAESG AECTNWQSSA 120
LAQKPYSGRR PDAIRLGLGN HNYCRNPDRD SKPWCYVFKA GKYSSEFCST PACSEGNSDC 180
YFGNGSAYRG THSLTESGAS CLPWNSMILI GKVYTAQNPS AQALGLGKHN YCRNPDGDAK 240
PWCHVLKNRR LTWEYCDVPS CSTCGLRQYS QPQFRIKGGL FADIASHPWQ AAIFAAAAAS 300
PGERFLCGGI LISSCWILSA AHCFQERFPP HHLTVILGRT YRVVPGEEEQ KFEVEKYIVH 360
KEFDDDTYDN DIALLQLKSD SSRCAQESSV VRTVCLPPAD LQLPDWTECE LSGYGKHEAL 420
SPFYSERLKE AHVRLYPSSR CTSQHLLNRT VTDNMLCAGD TRSGGPQANL HDACQGDSGG 480
PLVCLNDGRM TLVGIISWGL GCGQKDVPGV YTKVTNYLDW IRDNMRPPAP APAPAPAEAG 540
EECDCGSPEN PCCDAATCKL RPGAQCAEGL CCDQCRFKKK RTICRRAKGD RRDDRCTGQS 600
ADCPRNRLYG 610

Sequence Number (ID): 39
Length: 614
Molecule Type: AA
Features Location/Qualifiers:
 - source, 1..614
 > mol_type, protein
 > organism, synthetic construct
Residues:
SYQVICRDEK TQMIYQQHQS WLRPVLRSNR VEYCWCNSGR AQCHSVPVKS CSEPRCFNGG 60
TCQQALYFSD FVCQCPEGFA GKCCEIDTRA TCYEDQGISY RGNWSTAESG AECTNWQSSA 120
LAQKPYSGRR PDAIRLGLGN HNYCRNPDRD SKPWCYVFKA GKYSSEFCST PACSEGNSDC 180
YFGNGSAYRG THSLTESGAS CLPWNSMILI GKVYTAQNPS AQALGLGKHN YCRNPDGDAK 240
PWCHVLKNRR LTWEYCDVPS CSTCGLRQYS QPQFRIKGGL FADIASHPWQ AAIFAAAAAS 300
PGERFLCGGI LISSCWILSA AHCFQERFPP HHLTVILGRT YRVVPGEEEQ KFEVEKYIVH 360
KEFDDDTYDN DIALLQLKSD SSRCAQESSV VRTVCLPPAD LQLPDWTECE LSGYGKHEAL 420
SPFYSERLKE AHVRLYPSSR CTSQHLLNRT VTDNMLCAGD TRSGGPQANL HDACQGDSGG 480
PLVCLNDGRM TLVGIISWGL GCGQKDVPGV YTKVTNYLDW IRDNMRPPAP APAPAPAPAP 540
AEAGEECDCG SPENPCCDAA TCKLRPGAQC AEGLCCDQCR FKKKRTICRR AKGDRRDDRC 600
TGQSADCPRN RLYG 614

Sequence Number (ID): 40
Length: 615
Molecule Type: AA
Features Location/Qualifiers:
 - source, 1..615
 > mol_type, protein
 > organism, synthetic construct
Residues:
SYQVICRDEK TQMIYQQHQS WLRPVLRSNR VEYCWCNSGR AQCHSVPVKS CSEPRCFNGG 60
TCQQALYFSD FVCQCPEGFA GKCCEIDTRA TCYEDQGISY RGNWSTAESG AECTNWQSSA 120
LAQKPYSGRR PDAIRLGLGN HNYCRNPDRD SKPWCYVFKA GKYSSEFCST PACSEGNSDC 180
YFGNGSAYRG THSLTESGAS CLPWNSMILI GKVYTAQNPS AQALGLGKHN YCRNPDGDAK 240
PWCHVLKNRR LTWEYCDVPS CSTCGLRQYS QPQFRIKGGL FADIASHPWQ AAIFAAAAAS 300
PGERFLCGGI LISSCWILSA AHCFQERFPP HHLTVILGRT YRVVPGEEEQ KFEVEKYIVH 360
KEFDDDTYDN DIALLQLKSD SSRCAQESSV VRTVCLPPAD LQLPDWTECE LSGYGKHEAL 420
SPFYSERLKE AHVRLYPSSR CTSQHLLNRT VTDNMLCAGD TRSGGPQANL HDACQGDSGG 480
PLVCLNDGRM TLVGIISWGL GCGQKDVPGV YTKVTNYLDW IRDNMRPEAA AKGGGGSGGG 540
GSEAGEECDC GSPENPCCDA ATCKLRPGAQ CAEGLCCDQC RFKKKRTICR RAKGDRRDDR 600
CTGQSADCPR NRLYG 615

Sequence Number (ID): 41
Length: 615
Molecule Type: AA

Features Location/Qualifiers:
- source, 1..615
> mol_type, protein
> organism, synthetic construct
Residues:
SYQVICRDEK TQMIYQQHQS WLRPVLRSNR VEYCWCNSGR AQCHSVPVKS CSEPRCFNGG 60
TCQQALYFSD FVCQCPEGFA GKCCEIDTRA TCYEDQGISY RGNWSTAESG AECTNWQSSA 120
LAQKPYSGRR PDAIRLGLGN HNYCRNPDRD SKPWCYVFKA GKYSSEFCST PACSEGNSDC 180
YFGNGSAYRG THSLTESGAS CLPWNSMILI GKVYTAQNPS AQALGLGKHN YCRNPDGDAK 240
PWCHVLKNRR LTWEYCDVPS CSTCGLRQYS QPQFRIKGGL FADIASHPWQ AAIFAAAAAS 300
PGERFLCGGI LISSCWILSA AHCFQERFPP HHLTVILGRT YRVVPGEEEQ KFEVEKYIVH 360
KEFDDDTYDN DIALLQLKSD SSRCAQESSV VRTVCLPPAD LQLPDWTECE LSGYGKHEAL 420
SPFYSERLKE AHVRLYPSSR CTSQHLLNRT VTDNMLCAGD TRSGGPQANL HDACQGDSGG 480
PLVCLNDGRM TLVGIISWGL GCGQKDVPGV YTKVTNYLDW IRDNMRPEAA AKEAAAKEAA 540
AKEAGEECDC GSPENPCCDA ATCKLRPGAQ CAEGLCCDQC RFKKKRTICR RAKGDRRDDR 600
CTGQSADCPR NRLYG 615

Sequence Number (ID): 42
Length: 610
Molecule Type: AA
Features Location/Qualifiers:
- source, 1..610
> mol_type, protein
> organism, synthetic construct
Residues:
EAGEECDCGS PENPCCDAAT CKLRPGAQCA EGLCCDQCRF KKKRTICRRA KGDRRDDRCT 60
GQSADCPRNR LYGPAPAPAP APASYQVICR DEKTQMIYQQ HQSWLRPVLR SNRVEYCWCN 120
SGRAQCHSVP VKSCSEPRCF NGGTCQQALY FSDFVCQCPE GFAGKCCEID TRATCYEDQG 180
ISYRGNWSTA ESGAECTNWQ SSALAQKPYS GRRPDAIRLG LGNHNYCRNP DRDSKPWCYV 240
FKAGKYSSEF CSTPACSEGN SDCYFGNGSA YRGTHSLTES GASCLPWNSM ILIGKVYTAQ 300
NPSAQALGLG KHNYCRNPDG DAKPWCHVLK NRRLTWEYCD VPSCSTCGLR QYSQPQFRIK 360
GGLFADIASH PWQAAIFAAA AASPGERFLC GGILISSCWI LSAAHCFQER FPPHHLTVIL 420
GRTYRVVPGE EEQKFEVEKY IVHKEFDDDT YDNDIALLQL KSDSSRCAQE SSVVRTVCLP 480
PADLQLPDWT ECELSGYGKH EALSPFYSER LKEAHVRLYP SSRCTSQHLL NRTVTDNMLC 540
AGDTRSGGPQ ANLHDACQGD SGGPLVCLND GRMTLVGIIS WGLGCGQKDV PGVYTKVTNY 600
LDWIRDNMRP 610
END

## Claims

1. A fusion protein, comprising:
(a) a tissue plasminogen activator or a variant thereof;
(b) a disintegrin or a variant thereof; and
(c) a linker positioned between the tissue plasminogen activator or the variant thereof and the disintegrin or the variant thereof and the linker having an amino acid sequence selected from SEQ ID Nos.: 1-7.

2. The fusion protein as claimed in claim 1, wherein a C-terminus of the tissue plasminogen activator or the variant thereof is linked to a N-terminus of the linker, and a N-terminus of the disintegrin or the variant thereof is linked to a C-terminus of the linker; or a C-terminus of the disintegrin or the variant thereof is linked to a N-terminus of the linker, and a N-terminus of the tissue plasminogen activator or the variant thereof is linked to a C-terminus of the linker.

3. The fusion protein as claimed in claim 1, wherein the tissue plasminogen activator is alteplase, reteplase, or tenecteplase.

4. The fusion protein as claimed in claim 1, wherein the tissue plasminogen activator has an amino acid sequence selected from SEQ ID Nos.: 8-10.

5. The fusion protein as claimed in claim 1, wherein the disintegrin is albolabrin, applagin, basilicin, batroxostatin, bitistatin, cereberin, cerastin, crotatroxin, durissin, elegantin, eristicophin, flavoridin, flavostatin, halysin, halystatin, jararacin, jarastatin, kistrin, lachesin, lutosin, molossin, rhodostomin, salmosin, saxatilin, tergeminin, trimestatin, trimucrin, trimutase, ussuristatin, or viridian.

6. The fusion protein as claimed in claim 1, wherein the disintegrin variant includes:
(a) a linking region having an amino acid sequence selected from SEQ ID Nos.: 11-15;
(b) a RGD motif having an amino acid sequence selected from SEQ ID Nos.: 16-28; and
(c) a C-terminal region having an amino acid sequence selected from SEQ ID Nos.: 29-33.

7. The fusion protein as claimed in claim 1, wherein the disintegrin variant has an amino acid sequence of SEQ ID No.: 34.

8. The fusion protein as claimed in claim 1, comprising an amino acid sequence selected from SEQ ID Nos.: 35-42.

9. A pharmaceutical composition, comprising:
a fusion protein as claimed in one of claims 1-8; and
a pharmaceutically acceptable carrier.

10. A use of a pharmaceutical composition as claimed in claim 9 for manufacturing a medicine for treating or preventing a disease related to thrombosis under the condition of a low bleeding risk.
